(19)

Europäisches
Patentamt
European
Patent Office
Office européen
des brevets

(11)     **EP 4 137 153 A1**

(12)     **EUROPEAN PATENT APPLICATION**

(43) Date of publication:
**22.02.2023   Bulletin 2023/08**

(21) Application number: **21191940.2**

(22) Date of filing: **18.08.2021**

(51) International Patent Classification (IPC):
**A61K 39/12** *(2006.01)*      **A61P 31/20** *(2006.01)*
**A61P 35/00** *(2006.01)*      **C07K 14/005** *(2006.01)*

(52) Cooperative Patent Classification (CPC):
**A61K 39/12; A61P 31/20; A61P 35/00;**
**C07K 14/005;** A61K 2039/5256; A61K 2039/53;
A61K 2039/585; A61K 2039/70; C12N 2710/10343;
C12N 2710/20022; C12N 2710/20034

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**
Designated Extension States:
**BA ME**
Designated Validation States:
**KH MA MD TN**

(71) Applicants:
- **Sirion Biotech GmbH**
  **82152 Planegg/Martinsried (DE)**
- **Universität Regensburg**
  **93040 Regensburg (DE)**
- **InProTher ApS**
  **2200 Copenhagen (DK)**
- **ProBioGen AG**
  **13086 Berlin (DE)**

(72) Inventors:
- **Thirion, Christian**
  **80337 München (DE)**
- **Pertl, Cordula**
  **80939 München (DE)**
- **Karlas, Alexander**
  **10245 Berlin (DE)**
- **Sandig, Volker**
  **13158 Berlin (DE)**
- **Jordan, Ingo**
  **13156 Berlin (DE)**
- **Holst, Peter Johannes**
  **2860 Søborg (DK)**
- **Rahbæk Boilesen, Ditte**
  **2100 Copenhagen (DK)**
- **Wagner, Ralf**
  **83623 Dietramszell (DE)**
- **Neckermann, Patrick**
  **93053 Regensburg (DE)**
- **Asbach, Benedikt**
  **93051 Regensburg (DE)**

(74) Representative: **Dentons Patent Solutions**
**Rechtsanwaltsgesellschaft mbH**
**Jungfernturmstraße 2**
**80333 München (DE)**

Remarks:
The complete document including Reference
Table(s) and the Sequence Listing(s) can be
downloaded from the EPO website

(54)     **THERAPEUTIC PAPILLOMA VIRUS VACCINES**

(57)     The present invention relates to a nucleic acid comprising or consisting of nucleic acid sequences encoding papilloma virus proteins E1, E6, and E7; wherein said nucleic acid molecule encodes a single polyprotein.

**EP 4 137 153 A1**

**Description**

The present invention relates to a nucleic acid comprising or consisting of nucleic acid sequences encoding papilloma virus proteins E1, E6, and E7; wherein said nucleic acid molecule encodes a single polyprotein.

In this specification, a number of documents including patent applications and manufacturer's manuals are cited. The disclosure of these documents, while not considered relevant for the patentability of this invention, is herewith incorporated by reference in its entirety. More specifically, all referenced documents are incorporated by reference to the same extent as if each individual document was specifically and individually indicated to be incorporated by reference.

Despite efficient prophylactic vaccines and the possibility to screen for cervical lesions, infection with human papillomavirus (HPV) was still responsible for more than 340.000 cervical cancer-related deaths worldwide in 2020 (Sung, H. et al., Global Cancer Statistics 2020: GLOBOCAN Estimates of Incidence and Mortality Worldwide for 36 Cancers in 185 Countries, CA: A Cancer Journal for Clinicians, 71(3), 209-249, 2001).

More than 85% of these cases occurred in middle- and low-income countries (Haghshenas, M. et al. Prevalence and type distribution of high-risk human papillomavirus in patients with cervical cancer: a population-based study. Infect. Agent. Cancer 8, 20, 2013). Currently, there are three approved prophylactic vaccines providing near complete protection against vaccine-targeted HPV types, yet vaccine uptake is incomplete (Brotherton, J. M. L., Zuber, P. L. F. & Bloem, P. J. N. Primary Prevention of HPV through Vaccination: Update on the Current Global Status. Curr. Obstet. Gynecol. Rep. 5, 210-224, 2016). Moreover, these vaccines do not lead to the eradication of pre-infected cells, since they target the major capsid protein L1, which is not expressed in infected basal layer- and cervical cancer cells (Lehtinen, M. et al. Overall efficacy of HPV-16/18 AS04-adjuvanted vaccine against grade 3 or greater cervical intraepithelial neoplasia: 4-year end-of-study analysis of the randomised, double-blind PATRICIA trial. Lancet. Oncol. 13, 89-99, 2012; Hildesheim, A. et al. Effect of human papillomavirus 16/18 L1 viruslike particle vaccine among young women with preexisting infection: a randomized trial. JAMA 298, 743-753, 2007; Schiller, J. T., Castellsagué, X. & Garland, S. M. A review of clinical trials of human papillomavirus prophylactic vaccines. Vaccine 30 Suppl 5, F123-38; 2012; Hung, C.-F., Ma, B., Monie, A., Tsen, S.-W. & Wu, T.-C. Therapeutic human papillomavirus vaccines: current clinical trials and future directions. Expert Opin. Biol. Ther. 8, 421-439, 2008). Thus, novel interventions such as therapeutic vaccines are desirable.

Whereas most HPV infections are spontaneously cleared within months, some persist for years (Ho, G. Y., Bierman, R., Beardsley, L., Chang, C. J. & Burk, R. D. Natural history of cervicovaginal papillomavirus infection in young women. N. Engl. J. Med. 338, 423-428,1998). These can progress towards low-grade squamous intraepithelial lesions (LSIL), which can further progress to high-grade squamous intraepithelial lesions (HSIL) and cervical cancer. The expression pattern of viral proteins changes during progression of SILs: in LSIL, mainly the early proteins E1/E2 are expressed, whereas in HSIL and transformed cells, E6/E7 are highly expressed and E2 expression is low or absent (Xue, Y. et al. HPV16 E2 is an immediate early marker of viral infection, preceding E7 expression in precursor structures of cervical carcinoma. Cancer Res. 70, 5316-5325, 2010; Chang, L., He, X., Yu, G. & Wu, Y. Effectiveness of HPV 16 viral load and the E2/E6 ratio for the prediction of cervical cancer risk among Chinese women. J. Med. Virol. 85, 646-654, 2013).

In many cases of SIL, however, natural regression occurs as a result of CD4$^+$ and CD8$^+$ T cell infiltration (Hibma, M. H. The Immune Response to Papillomavirus During Infection Persistence and Regression. The Open Virology Journal vol. 6, 2012). It has been shown that CD4$^+$ T cells play a major role in lesion regression with increased CD4:CD8 ratios being found in the stroma of LSIL (Monnier-Benoit, S. et al. Immunohistochemical analysis of CD4+ and CD8+ T-cell subsets in high risk human papillomavirus-associated pre-malignant and malignant lesions of the uterine cervix. Gynecol. Oncol. 102,22-31,2006). Genetic studies have shown that resistance and susceptibility loci for chronic infections and cancer cluster in the MHC II region. Furthermore, HPV16$^+$ cervical cancer patients have impaired memory CD4$^+$ T-helper responses against E2 and E6, which emphasizes the important role of T cell responses in preventing progression and clearing lesions (Brotherton et al, 2016; Lehtinen et al, 2012; Hildesheim et al, 2007; Schiller et al, 2012; Hung et al, 2008; Ho et al, 1998; Xue et al, 2010; Chang et al, 2013; Hibma, 2012; Monnier-Benoit et al, 2006; de Jong, A. et al. Human Papillomavirus Type 16-Positive Cervical Cancer Is Associated with Impaired CD4+ T-Cell Immunity against Early Antigens E2 and E6. Cancer Res. 64, 5449 LP — 5455; 2004; Woo, Y. L. et al. A prospective study on the natural course of low-grade squamous intraepithelial lesions and the presence of HPV16 E2-, E6- and E7-specific T-cell responses. Int. J. cancer 126, 133-141, 2010; Ragonnaud, E., Pedersen, A. G. & Holst, P. J. Breadth of T Cell Responses After Immunization with Adenovirus Vectors Encoding Ancestral Antigens or Polyvalent Papillomavirus Antigens. Scand. J. Immunol. 85, 182-190, 2017; Dillon, S. et al. Resolution of cervical dysplasia is associated with T-cell proliferative responses to human papillomavirus type 16 E2. J. Gen. Virol. 88, 803-813, 2007). It was also reported that HPV-exposed children have E2-specific T cell responses after clearing the infection (Koskimaa, H. M. et al. Human papillomavirus 16-specific cell-mediated immunity in children born to mothers with incident cervical intraepithelial neoplasia (CIN) and to those constantly HPV negative. J. Transl. Med. 13, 1-11, 2015). Yet, current research on therapeutic HPV vaccines is primarily focused on the HSIL and cancer-stage of the disease, and directed toward the oncogenic viral proteins E6 and E7 (Hung et al, 2008). However, targeting the infection prior to carcinogenesis could be advantageous in terms of reducing morbidity and suffering related to cancer treatment, and might be easier to achieve. Thus, to target pre-malignant infection, other early proteins should be included as antigens.

There is no suitable animal model to study persistent HPV infections in a preclinical setting, but *Macaca fascicularis* papillomavirus type 3 (MfPV3) has a close phylogenetic and phenotypic relationship to HPV16 (Ragonnaud et al, 2017; Chen, Z. et al. Non-human Primate Papillomaviruses Share Similar Evolutionary Histories and Niche Adaptation as the Human Counterparts. Front. Microbiol. 10, 2093, 2019). Naturally occurring infections with this virus are associated with long-term persistence and at least LSIL-like lesions in the cervix of breeding female cynomolgus macaques (*Macaca fascicularis*), making them an ideal non-human primate (NHP) animal model (Chen, Z. et al. Genomic diversity and interspecies host infection of alpha12 Macaca fascicularis papillomaviruses (MfPVs). Virology 393, 304-310, 2009; Wood, C. E., Chen, Z., Cline, J. M., Miller, B. E. & Burk, R. D. Characterization and Experimental Transmission of an Oncogenic Papillomavirus in Female Macaques. J. Virol. 81, 6339-6345 (2007).

In view of the deficiencies of the prior art, the technical problem underlying the present invention can be seen in the provision of improved means and methods for the eradication of papillomavirus as well as the prevention and treatment of papilloma virus-associated disorders. This technical problem is solved by the subject-matter of the enclosed claims.

In the first aspect, the present invention provides a nucleic acid comprising or consisting of nucleic acid sequences encoding papilloma virus proteins E1, E6, and E7; wherein said nucleic acid molecule encodes a single polyprotein.

The nucleic acid in accordance with the first aspect requires presence of sequences encoding three papilloma virus proteins. Preferred is that either exactly three or exactly four papilloma virus proteins are encoded. To the extent a fourth protein is encoded, preference is given to a further protein of the E family of papilloma virus proteins, in particular to E2. Preferred papillomavirus species and strains are disclosed further below. Particularly preferred is human papillomavirus 16 (HPV16).

In accordance with the invention, coding sequences encoding the corresponding full-length or substantially full-length proteins are employed. The concomitant presence of three or four full-length protein coding sequences provides for the presentation of a multitude of immunogenic epitopes in an organism vaccinated with the nucleic acid of the first aspect or a vector or plasmid comprising said nucleic acid. The term "substantially full-length" as used herein refers to the optional absence of up to 20, up to 15, up to 10, or up to 9, 8, 7, 6, 5, 4, 3, or up to 2, or 1 amino acid. Preferably these deletions are at the C-terminus, N-terminus and/or in regions where deletions do not remove immunogenic epitopes or do not affect the immunogenicity of epitopes present in other parts of the amino acid sequence of the encoded protein. Particularly preferred is that said deletions reduce the oncogenic potential of a papillomavirus protein. A preferred embodiment of the latter is detailed further below (C-terminal modification of E6). It is furthermore preferred that "substantially full-length" applies to E6 only, whereas E1, E7, and, to the extent present E2, are full-length.

Immune responses such as T cells directed against three or more, preferably four different full-length proteins will unfold synergistic activity against papilloma virus infections and disorders associated therewith. In particular, successful stimulation of E1/E2-specific cellular immunity primarily clears infections in the LSIL-stage, whereas E6/E7-specific responses mainly targets HSIL and cancer stages.

Further advantages of the invention include the surprising capability to completely eradicate a papillomavirus infection, and the usefulness of said nucleic acid for not only prophylactic, but also therapeutic purposes. This is particularly advantageous in view of the oncogenic potential of papillomavirus.

The approach of using full-length coding sequences outperforms prior art vaccines which employed sequences encoding only fragments as opposed to full-length proteins. In particular, the synergistic effect arising from the concomitant expression of three or four full-length viral proteins is superior to the concomitant use of only fragments of a plurality of proteins *inter alia* owing to the larger number of immunogenic epitopes being provided. On the other hand, the use of full-length proteins for immunization in many instances will require further measures to the extent a protein with oncogenic potential is among said proteins. Such measures are subject of certain embodiments disclosed further below.

The papillomavirus antigens of the invention, i.e., E1, E6, E7, and, to the extent present, E2, are encoded by the nucleic acid of the invention as a single polyprotein. In other words, the coding sequences therefor are part of a single open reading frame. The term "polyprotein" includes fusions of said antigens, more specifically direct fusions with no intervening amino acids between the sequences of the respective proteins. The term "polyprotein" also embraces proteins wherein one or more amino acids are inserted between the sequences of two neighboring proteins or placed N-terminal of the most N-Terminal E protein, or placed C-terminal of the most C-terminal E protein. Preferred implementations of such inserted sequences are given below and may serve to provide specific functions such as adjuvant function or self-cleaving function, and/or avoid structural interference between adjacent proteins. The latter issue may be addressed by sequences encoding short flexible linkers being located between the nucleic acid sequences encoding the antigens.

The notion of a single polyprotein being encoded by the nucleic acid of the invention does not exclude that such polyprotein, either during translation or once translation is completed, gives rise to more than one protein molecule.

The latter applies in particular to those embodiments of the nucleic acid of the invention where, in addition to said papillomavirus proteins, a self-cleaving peptide is encoded.

The term "nucleic acid" refers to a polycondensate of nucleotides. Preference is given to nucleic acids which are recognized and processed by polymerases and/or a ribosome. Preferred implementations are DNA and RNA; for details see further below. Albeit less preferred, the term "nucleic acid" also extends to polycondensates comprising one or more modified nucleotides. Sites of modification in a nucleotide are those well known in the art, i.e., the sugar, the phosphate and the base. Preferred modification in that respect are 2' modifications such 2' O-alkyl including 2' OMe and 2' halogen substitutions such as 2' F. Preferred phosphate modifications include thiophosphate. A preferred modified base is pseudouridine; see, for example, Nance and Meier, ACS Cent Sci. 2021 May 26; 7(5): 748-756. The mentioned base, sugar and/or phosphate modifications are especially preferred in the context of an RNA vaccine, i.e., where the nucleic acid of the invention is implemented as RNA.

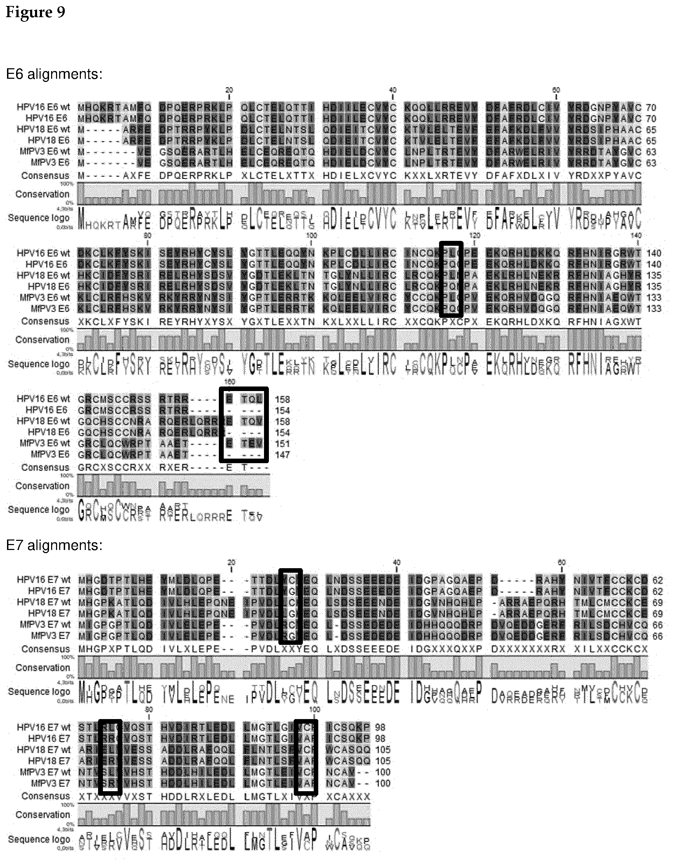

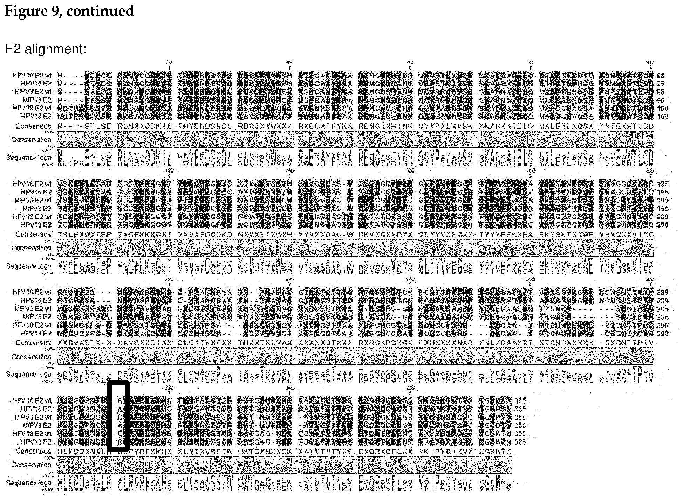

The E family of papillomavirus proteins are proteins referred to as "early" in the lifecycle of the virus. In functional terms, E1 has enzymatic function; it is an ATP-dependent helicase; see, e.g., Bergvall et al., Virology 2013 Oct;445(1-2):35-56. E6 is capable of inducing cell division in resting cells. It inhibits apoptosis and the immune response; see, e.g. Vande Pol and Klingelhutz, Virology 2013 Oct;445(1-2):115-37. Together with E7 it has transforming effect. E7 is involved in replication of the viral genome (see, e.g., Roman and Munger, Virology 2013 445: 138-68) and, together with E6, transforms cells. Figure 9 displays a multiple sequence alignment of wild-type E proteins with preferred E proteins of the invention.

In a preferred embodiment, said nucleic acid furthermore comprises or further consists of a nucleic acid sequence encoding papilloma virus protein E2.

E2 is multifunctional and mainly involved in genome replication and transcription; see, e.g. McBride, Virology. 2013 Oct;445(1-2):57-79.

The term "further consists of" refers to a closed listing of a preceding aspect or embodiment and provides for an expansion of said closed list by addition of an explicitly recited feature, wherein the listing remains closed and confined to the features of said preceding aspect or embodiment, augmented by said explicitly recited additional feature.

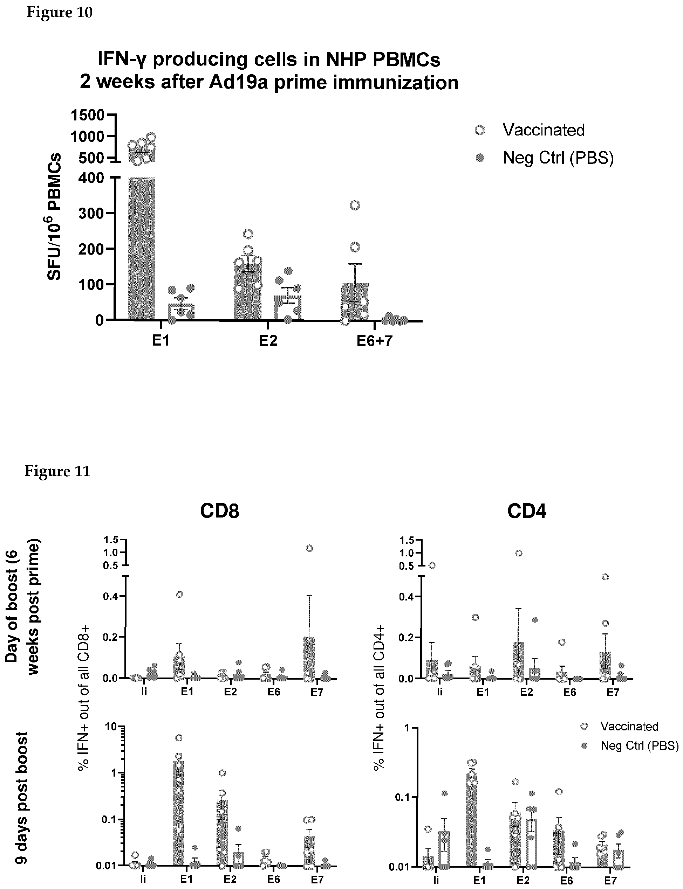

Of note, adding E2 as a further antigen does not deteriorate immune response; see, e.g. Example 4 and Figure 10.

Antigen sequences in accordance with the invention are given in the sequence listing as follows:

Amino acid sequences of E1, E2, E6 and E7 of MfPV3 are shown in SEQ ID NOs: 2, 4, 6, and 8, respectively.
Amino acid sequences of E1, E2, E6 and E7 of HPV16 are shown in SEQ ID NOs: 10, 12, 14, and 16, respectively.
Amino acid sequences of E1, E2, E6 and E7 of HPV18 are shown in SEQ ID NOs: 18, 20, 22, and 24, respectively.

The encoding nucleic acids for these proteins are shown, in the same order, in SEQ ID NOs: 1, 3, 5, 7, 9, 11, 13, 15, 17, 19, 21, and 23, respectively.

In accordance with said preferred embodiment, the nucleic acid of the invention encodes the four proteins E1, E2, E6 and E7 as a polyprotein, wherein preferably no further proteins of papillomavirus origin are encoded by said nucleic acid.

In a further preferred embodiment, the order of the nucleic acids encoding said proteins is, from 5' to 3', (a) to the extent E2 is present (i) E1, E2, E6, E7 or (ii) E1, E6, E7, E2; or (b) E1, E6, E7. Preferably, any of these listings is closed, i.e., no further sequence encoding a further protein of papillomavirus origin is present. The embodiment on the other hand extends to implementations where further amino acid sequences, preferably functional amino acid sequences, are present (for details see further below).

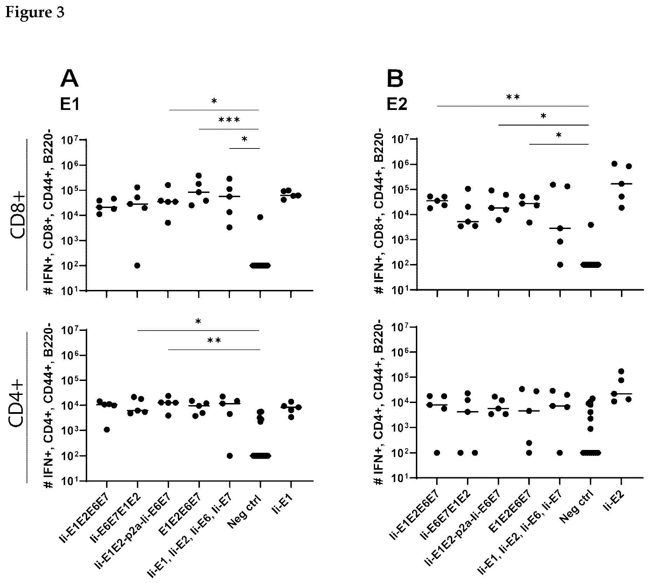

Most preferred is that sequences encoding the four proteins E1, E2, E6 and E7 are present and in the indicated order (from 5' to 3'). While also constructs of the invention with a different order perform well, it can be seen e.g. in Figure 3B that the order in the most preferred construct secures even better performance in terms of the E2 response.

In a further preferred embodiment, said nucleic acid of the first aspect comprises nucleic acid sequences encoding papilloma virus proteins E6 and/or E7, wherein said nucleic acid sequences are modified as compared to the wild-type counterparts by point mutations and/or deletions, wherein said point mutations and/or deletions (i) abolish colony forming in a soft agar assay, at least to a background level; and (ii) said modified proteins E6 and E7 maintain at least 70 % of predicted high-affinity epitopes ($IC_{50} \leq 50$ nM) and at least 50 %, at least 55% or at least 58% of predicted low-affinity epitopes (50 nM < $IC_{50} \leq 5\ \mu$M) of the predicted epitopes of the respective wild-type protein, preferably in terms of (predicted) binding to the most frequent HLA-A, -B, and -C alleles which preferably together comprise 75 % of the human HLA diversity for each HLA, respectively. This preferred embodiment allows for the presence of further papillomavirus proteins, E1 and optionally E2 being preferred in this context.

Said most frequent alleles are as follows:

HLA-A*01:01

HLA-A*02:01
HLA-A*02:02
HLA-A*02:03
HLA-A*02:05
HLA-A*02:06
HLA-A*02:07
HLA-A*02:11
HLA-A*02:12
HLA-A*02:16
HLA-A*02:17
HLA-A*02:19
HLA-A*02:50
HLA-A*03:01
HLA-A*11:01
HLA-A*24:02
HLA-A*24:03
HLA-A*30:01
HLA-A*30:02
HLA-A*33:01
HLA-B*07:02
HLA-B*08:01
HLA-B*08:02
HLA-B*08:03
HLA-B*15:01
HLA-B*15:02
HLA-B*15:03
HLA-B*15:09
HLA-B*15:17
HLA-B*18:01
HLA-B*27:05
HLA-B*27:20
HLA-B*35:01
HLA-B*35:03
HLA-B*39:01
HLA-B*40:01
HLA-B*40:02
HLA-B*40:13
HLA-B*44:02
HLA-B*44:03
HLA-B*51:01
HLA-B*58:01
HLA-B*58:02
HLA-C*03:03
HLA-C*04:01
HLA-C*06:02
HLA-C*07:01
HLA-C*07:02
HLA-C*08:02

In a particularly preferred embodiment, said modified E6 protein maintains at least 80%, at least 90%, at least 95%, or at least 98% of predicted high-affinity epitopes ($IC_{50} \leq 50$ nM) and at least 60 %, at least 70%, at least 80%, at least 90%, or at least 93% of predicted low-affinity epitopes (50 nM $< IC_{50} \leq 5$ $\mu$M) of the predicted epitopes of wild-type E6, preferably determined as specified above.

Related thereto, and in a separate aspect, the present invention provides a nucleic acid comprising or consisting of nucleic acid sequences encoding papilloma virus proteins E6 and/or E7, wherein said nucleic acid sequences are modified as compared to the wild-type counterparts by point mutations and/or deletions, wherein said point mutations and/or deletions (i) abolish colony forming in a soft agar assay; and (ii) said modified proteins E6 and E7 maintain at least 70 % of predicted high-affinity epitopes ($IC_{50} \leq 50$ nM) and at least 50 %, at least 55% or at least 58% of predicted low-affinity epitopes (50 nM < $IC_{50} \leq 5 \mu M$) of the predicted epitopes of the respective wild-type protein, preferably in terms of binding to the most frequent HLA-A, -B, and -C alleles which preferably together comprise 75 % of the human HLA diversity for each HLA, respectively. In a preferred embodiment of said separate aspect, said modified E6 protein maintains at least 80%, at least 90%, at least 95%, or at least 98% of predicted high-affinity epitopes ($IC_{50} \leq 50$ nM) and at least 60 %, at least 70%, at least 80%, at least 90%, or at least 93% of predicted low-affinity epitopes (50 nM < $IC_{50} \leq 5 \mu M$) of the predicted epitopes of wild-type E6, preferably determined as specified above. This aspect does not require any further nucleic acid sequences encoding papilloma virus proteins to be present.

As noted above, proteins E6 and E7 play a role in controlling and modifying host cell behaviour and can unfold oncogenic or transforming potential. As a consequence, using these proteins as immunogens is desirable and attractive, especially when it comes to control, eradicate, prevent, mitigate or treat papillomavirus-associated malignant disorders. On the other hand, their oncogenic potential entails corresponding risks associated with their use as a vaccine or components of a vaccine. In line with these considerations, the above preferred embodiment provides measures to mitigate or abolish said oncogenic potential. In this context, a balance is struck by said embodiment in that oncogenicity is controlled while immunogenicity is retained. Preferably, said point mutations and deletions are minimal in number.

As a measure of oncogenic potential, the art-established standard which is a soft agar assay is used; see also the enclosed Examples.

As a measure of immunogenicity, *in silico* methods predicting immunogenicity and/or the number of immunogenic epitopes are used. Such methods include computer algorithms that predict T cell epitopes binding to MHC I molecules. A preferred *in silico* method in this respect is the IEDB analysis resource ANN (also known as NetMHC), ver 4.0 (Nielsen et al., Protein Sci 12: 1007 (2003); Lundegaard et al., Nucl Acids Res 36, W509 (2008); Andreatta et al., Bioinformatics 32, 511 (2016)). Preferred is also that at least 50%, at least 60%, at least 70%, at least 75%, at least 80%, at least 90%, at least 95%, at least 98% or all immunogenic epitopes as they are present in the corresponding wild-type proteins are retained in modified E6 and/or E7.

As will become apparent in the following, the present invention provides, in a preferred embodiment, a minimum of modifications as compared to the corresponding wild-type sequences, wherein said minimum achieves both the required performance in a soft agar assay as well as in terms of retaining a high percentage of the immunogenic epitopes of the corresponding wild-type sequences.

Accordingly, in a particularly preferred embodiment, only the following modifications as compared to the wild-type are present in the encoded proteins:

  (i) in E6: a modification which reduces or abolishes interactions mediated by the PDZ domain comprised in wild-type E6; and

    a. in HPV16 E6: $L117X_1$;
    b. in HPV18 E6: $L112X_1$;
    c. in MfPV3 E6: $L110X_1$;

  and

  (ii) in E7:

    a. in HPV16 E7: $C24X_2$, $L67X_3$, and $C91X_4$;
    b. in HPV18 E7: $C27X_2$, $L74X_3$, and $C98X_4$;
    c. in MfPV3 E7: $C24X_2$, $L71X_3$, and $C95X_4$;

wherein $X_1$ to $X_4$ are independently a proteinogenic amino acid different from the respective amino acid they substitute.

Generally speaking, a position in an E2, E6 or E7 protein from a given first papillomavirus species corresponding to a position in an E2, E6 or E7 protein, respectively, from a given second papillomavirus species can be determined using the multiple sequence alignment shown in Figure 9. Corresponding positions are one on top of the other. In other words, the determination of corresponding positions is straightforward in view of the high degree of conservation across different HPV strains, in particular using said multiple sequence alignment to which sequences from any further papillomavirus strain can be added without further ado. Such alignments can be prepared with computer programs well known to the skilled person, preferably with CLC Main Workbench version 8.0.1 (QIAGEN, Aarhus, Denmark) using the default settings (very accurate; gap open cost 10.0; gap extension cost 1.0, end gap cost: as any other).

Preferably, but not necessarily, said nucleic acid furthermore comprises one or both mutations encoding $D21X_5$ and $C58X_6$ in HPV 16 E7, $D24X_5$ and $C65X_6$ in HPV 18 E7, and $D21X_5$ and $C62X_6$ in MfPV3 E7, wherein $X_5$ and $X_6$ are independently a proteinogenic amino acid different from the respective amino acid they substitute.

In terms of specific modifications, it is preferred that (i) $X_1$ is selected from Q, Y, and N, and preferably is Q;

(ii) $X_2$ is selected from G, A, V, L, and I, and preferably is G; (iii) $X_3$ is selected from R, K, and H, and preferably is R; (iv) $X_4$ is selected from A, G, V, L, and I, and preferably is A; (v) $X_5$ is selected from G, A, V, L, and I, and preferably is G; (vi) $X_6$ is selected from G, A, V, L, and I, and preferably is G; and/or (vii) said modification which reduces or abolishes interactions mediated by the PDZ domain is a deletion of 1 to 10 C-terminal amino acids, preferably counted from the C-terminus, more preferably ΔETQL in HPV16 E6, ΔETQV in HPV18 E6, and ΔETEV in MfPV3 E6; or said modification are one, two, three or four point mutation with the 10, preferably the four C-terminal amino acids.

The notation "Δ" is art-established and, in the context of this embodiment, refers to the deletion of the four indicated C-terminal residues.

In a further preferred embodiment, the total number of Cys residues in said polyprotein is an even number. How this is achieved is not particularly limited. Preference is given to implementations which do not entail loss of one or more immunogenic epitopes. Without wishing to be bound by a specific theory, it is envisaged that an even number of Cys residues increases antigen yield. To explain further, antigens, when fused to invariant chain (for details regarding the invariant chain see further below) are directed to the secretory pathway. In the endoplasmic reticulum (ER), where chaperones (such as protein-disulfide-isomerase that binds to free cysteines) could detect free Cys which in turn might lead to an ER-stress-response that then would lead to a decrease or stop in protein-synthesis thus limiting expression of the antigen.

In a particularly preferred embodiment, if said total number of Cys residues is otherwise not even,

(i) a sequence encoding

    a. HPV16 E2 with a $C300X_7$ mutation;
    b. HPV18 E2 with a $C301X_7$ mutation; or
    c. MfPV3 E2 with a $C297X_7$ mutation
    is comprised in said nucleic acid as sequence encoding E2, wherein $X_7$ is a proteinogenic amino acid other than C, preferably selected from A, S, G, M, T, Y, Q, N, V, L, I, F, W, H, K, R, Q, N, and P, more preferably A;

(ii) or a codon encoding Cys is inserted in said nucleic acid.

In a further preferred embodiment, said nucleic acid furthermore comprises at least one nucleic acid sequence encoding a genetic adjuvant, preferably T cell adjuvant MHC-II-associated invariant chain. Said invariant chain is also abbreviated as "li" in the following. The T cell adjuvant MHC-II-associated invariant chain has successfully been used as a genetic adjuvant (i.e., an adjuvant encoded by a nucleic acid, said nucleic acid optionally comprising also nucleic acid sequences encoding the antigen(s) against which an immune response is to be raised) in several contexts, see, for example, published international patent applications WO 2007/062656, WO 2010/057501 and WO 2018/172259. In terms of the species said invariant chain originates from, preference is given to vertebrates, including teleost invariant chain (see, e.g. WO 2020/079234), shark invariant chain (see, e.g., WO 2015/082922) and mammalian, preferably human invariant chain; see the above cited three WO documents. Particularly preferred is human invariant chain.

Further genetic adjuvants which may be used in accordance with the invention are heat-shock proteins, calreticulin and C4b.

Presence of said invariant chain entails unexpected performance owing to a synergistic effect. In particular, the combination of the above disclosed modifications of the oncogenic proteins E6 and E7 with li reduces oncogenicity to an unexpected degree. Without wishing to be bound by a specific theory, this is considered to be a consequence of a technical effect provided by li: export of E6 and E7, as such and in the absence of a fused li predominantly localized in the nucleus, into the cytoplasm where the oncogenic potential cannot unfold or only to a lesser extent.

In a particularly preferred embodiment, said invariant chain (i) is present once and precedes the sequence encoding the first E protein; or (ii) is present twice, one copy preceding the sequence encoding the first E protein, and a second copy preceding the sequence encoding E6 or, to the extent present, the sequence encoding E2. This embodiment relates to exemplary or preferred constructs, where a specific placement of said invariant chain within a nucleic acid of the invention applies. While such exemplary embodiments have been reduced to practice (see the Examples enclosed herewith), the invention is not so limited. The term "first E protein" refers to the most N-terminal protein selected from E1, E6 and E7, and, if present, E2 in the fusion protein encoded by the nucleic acid of the invention. Preferred is that E1 is the first E protein.

As an alternative or in addition to said genetic adjuvant, adjuvants known in the art may be used. Such adjuvants include cytokines, adhesion molecules, chemokines, CCR1/5 agonists, MIP-1 alpha, danger signals, LPS and derivatives thereof such as lipid A and lipid A derivatives, and TLR agonists.

In a further preferred embodiment, said nucleic is (i) DNA; (ii) a plasmid; or (iii) RNA, preferably mRNA. mRNA vaccines are being developed and several have received market approval such as Comirnaty of Biontech/Pfizer and mRNA-1273 of Moderna Therapeutics. In view of its inherent instability, RNAs may be chemically modified in order to enhance their stability; see, e.g. the modifications described for siRNAs in Hu et al., Signal Transduction and Targeted Therapy 2020,5:101 as well the modifications disclosed herein further above. mRNA vaccines generally comprise a Kozak sequence at the translation start point, a translation termination codon at the end of the open reading frame, and a poly-adenylation site in the 3'-untranslated region.

In a preferred embodiment, said DNA and said plasmid comprise a promoter, preferably a promoter active in eukaryotic cells. Such promoters include a CMV promoter, preferably with a HTLV-1 enhancer element, UbC promoter, EF1alpha promoter and SV40 promoter.

In a preferred embodiment, said plasmid comprises a bacterial origin of replication such as pUC ori, and more preferably also selection marker such as an antibiotic resistance gene such as a Kanamycin resistance gene.

In a further preferred embodiment, said nucleic acid, in particular said DNA, comprises an untranslated 5' terminus comprising a Tet operator. Among the known Tet-Off and Tet-On systems, preference is given to Tet-Off, given that the off-switch would be a constituent of the producer cell and not of the virus product. A preferred Tet operator site is shown in SEQ ID NO: 105. The Tet system employed by the present invention is the system known as T-REx system.

In the T-REx system the gene of interest is flanked by an upstream CMV promoter and two copies of tetracycline operator 2 (TetO2) sites. Expression of the gene of interest is repressed by the high affinity binding of TetR homodimers to each TetO2 sequences in the absence of tetracycline. In the present invention the production cell lines were modified to express the Tet repressor TetR. Introduction of tetracycline results in binding of one tetracycline on each TetR homodimer followed by release of the TetR homodimer from the TetO2. Unbinding of TetR homodimers form the TetO2 results in de-repression of the gene of interest. See, for example, Hillen W. and Berens. Mechanisms underlying expression of Tn10 encoded tetracycline resistance, Annu Rev Microbiol. 1994;48:345-69; W Hillen, C Gatz, L Altschmied, K Schollmeier, I Meier. Control of expression of the Tn10-encoded tetracycline resistance genes. Equilibrium and kinetic investigation of the regulatory reactions, J Mol Biol. 1983 Sep 25;169(3):707-21; Kathleen Postle, Toai T. Nguyen, Kevin P. Bertrand. Nucleotide sequence of the repressor gene of the TN10 tetracycline resistance determinant. Nucleic Acids Research, Volume 12, Issue 12, 25 June 1984, Pages 4849—4863; and Feng Yao, Tor Svensjö, Thomas Winkler, Michael Lu, Carl Eriksson, and Elof Eriksson. Tetracycline Repressor, tetR, rather than the tetR-Mammalian Cell Transcription Factor Fusion Derivatives, Regulates Inducible Gene Expression in Mammalian Cells, Human Gene Therapy. Sep 1998.1939-1950..

In a preferred embodiment, said nucleic acid comprises at least one nucleic acid sequence encoding a self-cleaving peptide or a peptide which causes a ribosome not to form a peptide bond while continuing translation, wherein said nucleic acid sequence encoding said peptide preferably (i) precedes at least one nucleic acid sequence encoding a genetic adjuvant; or (ii) is located between and adjacent to two nucleic acid sequences encoding a papilloma virus protein.

Given that a maximum number of immunogenic epitopes is to be presented, it is not preferred to insert said nucleic acid sequence encoding a self-cleaving peptide into a nucleic acid sequence encoding one of the antigens of the invention.

A preferred peptide sequence is referred to as "p2a" herein.

Especially preferred nucleic acids in accordance with the invention are those which encode the following polyproteins:

li-E1E2E6E7
li-E1E2-p2a-li-E6E7
E1E2E6E7
li-E1E6E7-p2a-li-E2
li-E1E6E7

The sequences of nucleic acids encoding these constructs, wherein the encoded proteins are or are based on MfPV3 proteins are, in the same order: SEQ ID NOs: 25, 27, 29, 31 and 33, and the corresponding polyprotein sequences are shown in SEQ ID NOs: 26, 28, 30, 32 and 34, respectively.

The sequences of nucleic acids encoding these constructs, wherein the encoded proteins are or are based on HPV16 proteins are, in the same order: SEQ ID NOs: 35, 37, 39, 41 and 43, and the corresponding polyprotein sequences are shown in SEQ ID NOs: 36, 38, 40, 42 and 44, respectively.

The sequences of nucleic acids encoding these constructs, wherein the encoded proteins are or are based on HPV18 proteins are, in the same order: SEQ ID NOs: 45, 47, 49, 51 and 53, and the corresponding polyprotein sequences are shown in SEQ ID NOs: 46, 48, 50, 52 and 54, respectively.

SEQ ID NOs: 100 to 104 all relate to HPV18 li-E1E6E7, more specifically as follows: HPV18 liE1E6E7 nucleic acid sequence (SEQ ID NO: 100), HPV18 li-E1E6E7 protein sequence (SEQ ID NO: 101), HPV18 li-E1E6E7 payload as inserted into various viral vectors (SEQ ID NOs: 102 to 104).

Of note, to the extent E antigens are adjacent, they are preferably separated by a short flexible linker, preferably GS (see also below). A linker between li and an adjacent antigen is not required, but not excluded. A linker connecting p2a to any of the adjacent sequences, be it an antigen or li, is not required, but not excluded.

In a further preferred embodiment, said papilloma virus is a primate papilloma virus, preferably a human papilloma virus (HPV) such as HPV16, HPV18, HPV45, HPV31, HPV33, HPV35, HPV52, HPV58, HPV6 and HPV11, more preferably HPV16 or HPV18, or macaque papilloma virus, preferably MfPV3. Especially preferred is HPV16. Experimental evidence for HPV16 can be found in Example 4.

In a further preferred embodiment, neighbouring nucleic acid sequences are connected in one, more or all instances by sequences encoding a flexible linker which is 10 or less amino acids in length such as 2, 3, 4, or 5 amino acids, optionally comprising a Cys residue, wherein GS and GCS are preferred linkers. Preferably, such linkers are present only between neighbouring antigens; see also the discussion of the most preferred nucleic acids of the invention as given above.

A linker comprising a Cys residue such as GCS is preferably to be viewed in the context of the above disclosed preferred embodiment requiring an even number of Cys residues in the polyprotein of the invention. In other words, while a Cys-containing linker such as GCS is generally useful, it is considered to employ it in particular in those cases where the polyprotein encoded by the nucleic acid of the invention would otherwise have an uneven number of Cys residues. For example, use may be made of a GCS linker for connecting E1 and E6 such as in SEQ ID NOs: 100 to 104.

In a second aspect, the present invention provides a viral vector comprising the nucleic acid of any one of the preceding claims.

In a preferred embodiment, said vector is a DNA viral vector, preferably (i) an adenoviral vector, preferably being deficient with regard to adenoviral E1 and E3 encoding sequences, said vector more preferably belonging to class C, D or E and/or being Ad19a/64, Ad5, Ad5 with fiber replacements such as Ad5F35, Ad26, Chimp63, or chAdOx1; or (ii) a Poxvirus vector, preferably MVA, MVA CR19, SCV, Vaccinia, or Fowlpox.

Ad5 has been described in Schiedner G, Hertel S, Kochanek S. Hum Gene Ther. 2000 Oct 10;11(15):2105-16. doi: 10.1089/104303400750001417. PMID: 11044912.

Ad19a is also known as Ad64; see, for example, Zhou X., Robinson C.M., Rajaiya J., Dehghan S., Seto D., Jones M.S., Dyer D.W., Chodosh J. Analysis of human adenovirus type 19 associated with epidemic keratoconjuctivitis and its reclassification as adenovirus type 64, Invest. Ophthalmol. Vis. Sci, 53 (2012), pp. 2804-2811. Ad19a is also described in Ruzsics Z, Wagner M, Osterlehner A, Cook J, Koszinowski U, Burgert HG. J Virol. 2006 Aug;80(16):8100-13. doi: 10.1128/JVI.00687-06. PMID: 16873266.

Adenoviruses with fiber replacements are described, for example, in Shayakhmetov, D. M.; Papayannopoulou, T.; Stamatoyannopoulos, G.; Lieber, A. Efficient Gene Transfer into Human CD34+ Cells by a Retargeted Adenovirus Vector. J. Virol. 2000.

Ad5F35 specifically has been described in Flickinger Jr JC, Singh J, Carlson R, et al. Chimeric Ad5.F35 vector evades anti-adenovirus serotype 5 neutralization opposing GUCY2C-targeted antitumor immunity, Journal for Immuno-Therapy of Cancer. 2020. Here it is shown that Ad5 and Ad5F35 induces comparable immune responses and tumour control in naïve mice, and that Ad5F35 induces better responses than Ad5 in mice pre-exposed to Ad5 infection. Further, it was shown that fewer patients have neutralising antibodies against Ad5F35 compared to Ad5. As such, and in view of its superior performance, Ad5F35 is particularly preferred.

Particularly preferred DNA payloads (inserts; sequences comprising antigen-encoding sequences) in accordance with the invention for various viral vectors are shown in SEQ ID NOs: 55 to 99 and 102 to 104. For further information regarding the specific features of the respective vector, see the respective annotations in the sequence listing.

The procedure for producing adenoviral vectors with inserts is known in the art and described, for example, in Ruzsics Z, Lemnitzer F, Thirion C. Methods Mol Biol. 2014;1089:143-58. doi: 10.1007/978-1-62703-679-5_11. PMID: 24132484.

The sequences of MVA vectors can be found in sequence databases as follows:

MVA sequence: GenBank (release 244.0) accession number: U94848, version number: U94848.1, release date 14.04.2003, https://www.ncbi.nlm.nih.gov/nuccore/U94848.1;
MVA.CR19 sequence: GenBank (release 244.0) accession number: KY633487, version number KY633487.1, release date 28.03.2017, https://www.ncbi.nlm.nih.gov/nuccore/KY633487.1;

For the purpose of producing or multiplying said vector, a cell line, preferably a eukaryotic cell line may be employed, wherein said cell line comprises said vector. Suitable eukaryotic cell lines include HEK293, PerC6, AGE1.CR.pIX (Jordan I, Vos A, Beilfuss S, Neubert A, Breul S, & Sandig V, 2009. An avian cell line designed for production of highly attenuated viruses. Vaccine 27, 748-756. https://doi.org/10.1016/j.vaccine.2008.11.066. PMID 19071186), HEK293 T-REx and AGE1.CR.pIX-T-REx (derived from HEK293 and AGE1.CR.pIX, respectively, already expressing TetR), A549, and A549 with engineered adenovirus E1 overexpression. As noted above, to the extent not already present, said cell lines are preferably, but not necessarily modified to express TetR.

MVA is adapted to replication in avian cells. For production of MVA a host is therefore preferred such as primary chicken embryo fibroblasts (CEF) or AGE1.CR.pIX that is derived from duck retina cells. As opposed to primary cells, an immortalized (or continuous) cell line such as AGE1.CR.pIX has several advantages: the cell substrate can be retrieved out of locally stored cryocultures and thus is resilient to supply constraints. An immortal cell line can furthermore be characterized against adventitious agents at the level of the cell bank, well ahead of the actual production processes. The AGE1.CR.pIX cell line (as opposed to primary material) furthermore proliferates in suspension in media free of animal derived components. This property allows highly efficient and scalable fed-batch production processes for poxviruses of different genera (Jordan I, Northoff S, Thiele M, Hartmann S, Horn D, Höwing K, Bernhardt H, Oehmke S, von Horsten H, Rebeski D, Hinrichsen L, Zelnik V, Mueller W, & Sandig V, 2011. A chemically defined production process for highly attenuated poxviruses. Biologicals 39, 50-58. https://doi.org/10.1016/j.biologicals.2010.11.005. PMID 21237672).

Vaccinia viruses mature into infectious particles with one or three membranes without the requirement for budding. One consequence of this complex infectious cycle is that the majority of the wild-type virions remain associated with the producer cell and that only a small fraction of infectious activity can be measured in the culture supernatant. The development of the novel strain MVA-CR19 has been induced by this observation (Jordan I, Horn D, John K, & Sandig V, 2013. A genotype of modified vaccinia Ankara (MVA) that facilitates replication in suspension cultures in chemically defined medium. Viruses 5, 321-339. https://doi.org/10.3390/v5010321. PMID 23337383). MVA-CR19 is a strain of MVA with a unique genotype (Jordan I, Horn D, Thiele K, Haag L, Fiddeke K, & Sandig V, 2019. A Deleted Deletion Site in a New Vector Strain and Exceptional Genomic Stability of Plaque-Purified Modified Vaccinia Ankara (MVA). Virol Sin. https://doi.org/10.1007/s12250-019-00176-3. PMID 31833037). Point mutations in structural genes and recombination of a large portion of the inverted terminal repeat (ITR) at the left side of the linear genomic DNA have profound effects on the phenotype of MVA-CR19. For example, compared to wild-type, MVA-CR19 releases a larger number of infectious particles into the culture supernatant and replicates to higher infectious titers. Viral factors that impact immune responses of the host and the infectious cycle are encoded in the ITRs. The recombination event in MVA-CR19 has changed the expression pattern of these factors (some were deleted, for others the gene dosehas been duplicated) with positive effects on efficacy and stability as a vaccine vector.

The potentially enhanced release of MVA-CR19 from host cells can also be seen in the CPE in adherent cells: whereas wild-type MVA tends to induce cell fusion and syncytia with well circumscribed plaques, infection with MVA-CR19 leads to a pattern consisting of large but loosely packed (unfused) plaques surrounded by isolated infected cells scattered at greater distances to the primary plaque or localized in comets.

Generation and isolation of recombinant MVA is complex due to the large size of the viral genome (178 kb). The most commonly used technique relies on homologous recombination in infected host cells with a shuttle plasmid that contains the gene of interest. The recombinant viruses must be isolated and purified from a vast background of contaminating parental viruses without the insert. While MVA-CR19 has advantages for production and vaccine efficacy it can be more complex to purify due to the less confined nature of replication. Furthermore, for both wild-type and MVA-CR19, selection against expression and maintenance of a transgene may occur if the novel sequence impairs the infectious cycle.

In such case, a cell line constitutively expressing TetR and an expression unit containing copies of tetO can be utilized to minimize expression of the transgene during virus generation and production.

In a third aspect, the present invention provides a polyprotein encoded by the nucleic acid or the vector of any of the preceding claims or cleavage products derived therefrom.

To the extent reference is made to cleavage products, these are preferably cleavage products arising from the activity of a self-cleaving sequence which in accordance with a preferred embodiment disclosed above may be present in the polyprotein encoded by the nucleic acid of the invention. It is understood that cleavage does not affect the number of epitopes presented by the proteins encoded by the nucleic acid of the invention. As such, the cleavage site in the encoded polyprotein are preferably located between sequences of antigens.

In a fourth aspect, the present invention provides a pharmaceutical composition, preferably a vaccine, comprising or consisting of (i) a nucleic acid of the first aspect; (ii) a vector of the second aspect; (iii) a polyprotein or cleavage products thereof of the third aspect; and/or (iv) a cell transduced with the nucleic acid of (i) or the vector of (ii), wherein preferably said cell is an ex vivo or in vitro cell and/or a dendritic cell or a monocyte.

As regards item (iv), it is known in the art that cells may be transformed with an antigen-encoding construct, thereby obtaining a population of cells with prophylactic or therapeutic properties against a disorder associated with said antigen. An example in the field of cancer is Maeng et al., Journal of Clinical Oncology 37, no. 15_suppl (May 20, 2019) 2639-2639. Preferably, said cells are autologous cells from the individual to be treated. Especially preferred for item (iv), to the extent a vector is used, is Ad19a/64.

In terms of routes of administration, intramuscular, subcutaneous, intradermal, vaginal, rectal, and/or mucosal administration, including to mucosae of the genital tract are envisaged.

Formulations include gels, in particular for mucosal, preferably vaginal administration.

Excipients, diluents and conservants are well known to the skilled person.

In terms of dosages, $10^9$ to $10^{11}$ infectious units are preferred for adenoviral vectors, and $5 \times 10^6$ to $5 \times 10^8$ for poxviral vectors. Generally speaking, suitable dosages can be determined by a clinician or manufacturer without further ado in view of the present disclosure, possibly of clinical studies, and, where necessary, taking into account parameters such as weight, age, sex etc. of the individual to be vaccinated. Experience tells, though, that in the field of vaccines there is generally a one-fits-all dosage or dosage regimen.

In a preferred embodiment said vector, said nucleic acid, said polyprotein or said cell is the only pharmaceutically active agent.

In an alternative preferred embodiment, said pharmaceutical composition comprises two or more pharmaceutically active agents, wherein a second pharmaceutically active agent is selected from (i) a second nucleic acid of the first aspect; (ii) a second vector of the second aspect; (iii) a second polyprotein or cleavage products of the third aspect; (iv) a second cell as defined above; (v) a protein-based vaccine, preferably against papilloma virus; and (vi) a chemotherapeutic, preferably cisplatin. The Examples demonstrate a synergistic effect when the vaccine according to the invention is given together with cisplatin.

There are known papillomavirus vaccines which are proteins or protein-based. In accordance with item (v) above, such known vaccines may be combined with a vaccine in accordance with the present invention. Of note, also the present invention may be implemented, albeit less preferred, as a protein-based vaccine; see above item (iii).

Further agents for a combination therapy together with constructs (nucleic acids, vectors, polyproteins, cells) of the invention include antibodies, preferably monoclonal antibodies targeting an immune checkpoint, such checkpoints preferably being PD-1, PD-L1 and CTLA-4.

Generally speaking, the components of a combination therapy may be administered together or separate in any order, possibly following different dosage regimens.

In a particularly preferred embodiment, said pharmaceutical composition comprises two pharmaceutically active agents which are (i) an adenoviral vector as defined above; and a Poxvirus vector as defined above, wherein said two pharmaceutically active agents are preferably confectioned as two pharmaceutical compositions which may be administered in any order or concomitantly, preferably the pharmaceutical composition comprising said Poxvirus vector after said pharmaceutical composition comprising said adenoviral vector, preferably after at least 4 weeks have elapsed.

This preferred embodiment defines preferred prime/boost schemes in accordance with the invention. As such, it is preferred that said two distinct vectors are administered in a manner which allows time to elapse between the two administrations. Preferred time spans are at least 2 weeks, at least 3 weeks, at least 4 weeks, at least 5 weeks, at least 6 weeks, at least 7 weeks or at least 8 weeks. Preferred upper limits for said time span are 6 weeks, 7 weeks, 8 weeks, 3 months, 4 months, 5 months or 6 months.

In a fifth aspect, the present invention provides the nucleic acid, the vector, the polyprotein, or the cell of any one of the preceding claims for use in medicine.

Preferably, said vaccine is a therapeutic vaccine. Of note, the combination of full-length antigens in accordance with the invention provides for eradication of the virus. As such, said vaccine has not only prophylactic applications, but is also a therapeutic agent.

In a sixth aspect, the present invention provides the nucleic acid, the vector, the polyprotein, the cell, or the vaccine of any of the preceding claims for use in a method of treating, ameliorating or preventing papilloma virus infection or papilloma virus induced disorders such as warts, malignant cell changes or cancer. Infection and associated disorders can occur in a variety of sites, e.g. nasal-oral, skin, genitals (inner and outer). Some of the best known papillomavirus associates malignancies and cancers are low-grade squamous intraepithelial lesions (LSIL), high-grade squamous intraepithelial lesions (HSIL), nasal polyposis or papilloma virus-associated malignant conditions such as cervical cancer, head and neck squamous cell carcinoma (HNSCC), and oropharyngeal squamous cell carcinoma (OPSCC).

Generally speaking, it is understood that these disorders are papilloma-virus associated. Papillomavirus strains which are frequently involved as causative agents are known in the art and include HPV16, HPV18, HPV45, HPV31, HPV33, HPV35, HPV52, HPV58, HPV6 and HPV11. To give an example, nasal polyposis may arise from infections with HPV 6 or HPV 11.

Table 1 below provides an overview of HPV strains associated with several malignant and non-malignant disorders.

| HPV type | Cervical (DOI: 10.1056/NEJMoa021641) | HNSCC (DOI: 10.1016/j.ejca. 2020.04.027 or) | Other cancers | genital warts |
|---|---|---|---|---|
| HPV16 | 54.6% | 86.0% | | |
| HPV18 | 11.0% | 1.0% | | |

(continued)

| HPV type | Cervical (DOI: 10.1056/NEJMoa021641) | HNSCC (DOI: 10.1016/j.ejca. 2020.04.027 or) | Other cancers | genital warts |
|---|---|---|---|---|
| HPV45 | 4.4% | 0.3% | | |
| HPV31 | 3.4% | 0.3% | | |
| HPV33 | 2.0% | 7.4% | | |
| HPV35 | 2.0% | 3.4% (HPV35 is not in gardasil-9) | | |
| HPV52 | 2.2% | | | |
| HPV58 | 2.0% | 0.10% | | |
| HPV6 and 11 | 0.2% | HPV11 0.1% (7.8% in doi: 10.3390/jcm7090241) | | 90% (https://www.cancer.gov/) |
| | | | HPV (of any type) is found in 40% vulva, vaginal and penile cancer (doi: 10.3390/jcm7090241) | |

In a seventh aspect, the present invention provides an *in vitro* or *ex vivo* method of stimulating and/or expanding T cells, said method comprising bringing into contact *in vitro* or *ex vivo* T cells with a nucleic acid, a vector, or a polyprotein of any one of the preceding aspects of the invention.

In a preferred embodiment, said bringing into contact is effected in the presence of antigen-presenting cells. Such antigen-presenting cells are preferably dendritic cells, monocytes, macrophages or B lymphocytes. The presence of antigen-presenting cells provides for the expanded T cells to be MHC-restricted.

Preferred is that said T cells are *ex vivo,* i.e., obtained from an individual or patient. Envisaged is to put back said T cells, once they are expanded, into the same patient or individual. Such re-introduction of expanded T cells is also known in the art as adoptive immunotherapy.

Related thereto, the present invention provides in a further aspect T cells, preferably MHC-restricted T cells obtained by the method of the seventh aspect.

The Figures show:

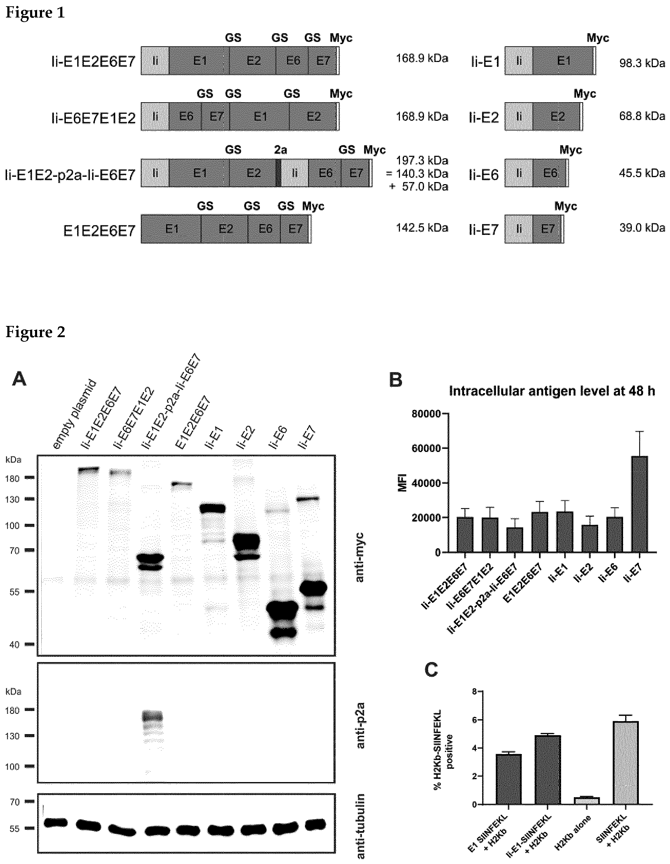

**Figure 1:** Schematic representation of the conceived MfPV3 antigen variants. MfPV3 antigens were designed as fusion proteins comprising either E1, E2, E6 and E7 altogether, or E1 plus E2 and E6 plus E7 fusion proteins linked by a p2a peptide, and fused to the MHC-II invariant chain (Ii), respectively. For reference, E1, E2, E6, and E7 were fused as single open reading frames without the Ii coding sequence. Theoretical molecular weight in kDa calculated from amino acid sequence. Abbreviations: Ii: MHC-II associated invariant chain, GS: glycine-serine-linker, 2a: p2a peptide for cotranslational separation, Myc: myc-tag sequence for antibody detection, kDa: kilo Dalton.

**Figure 2:** Expression analysis of MfPV3 antigens. A. Western blot analysis of HEK293T cell lysates 48 h following transfection with equimolar amounts of pURVac DNA vaccines expressing the various MfPV3 antigens. Antigens were detected with anti-myc (upper panel) and anti-p2a-peptide (middle panel) antibodies. Tubulin levels were monitored using an anti-tubulin antibody as loading control (lower panel). B. Flow cytometry analysis of HEK293T cells 48 hours following transfection with pURVac DNA vaccines. Intracellular staining was performed with anti-myc antibody. Depicted is the mean fluorescence intensity (MFI) of the average of 3 independent experiments. Error bars indicate standard error of the mean. C. Co-transfection of HEK293T cells with H2Kb alone or together with pURVac E1-SIINFEKL or Ii-E1-SIINFEKL, respectively, and analysis of SIINFEKL-H2Kb on cell surfaces 48 hours later by flow cytometry using an antibody recognizing the SIINFEKL-H2Kb-complex. Depicted is the percentage of H2Kb-SIINFEKL positive cells of the average of 6 independent experiments. Error bars indicate standard error of the mean. Light gray bars are negative and positive controls, respectively.

**Figure 3:** T cell responses induced by the various pURVac DNA vaccines encoding E1, E2, E6 and E7 in outbred CD1 mice. CD1 mice (5 per group) were immunized 4 times in 1 week intervals with 0.5 μg DNA of pURVac DNA encoding the indicated MfPV3 early antigens. Mice were sacrificed 7 days post last immunization, spleens were harvested and CD8 (top panels) and CD4 (bottom panels) T-cell immune responses against E1 (A), and E2 (B) were measured using ICS and flow cytometry. Negative control groups consist of all mice immunized with a pURVac DNA vaccine encoding antigens not covered by the peptide pools used for in vitro restimulation. Asterisks between groups indicate significant differences in response-levels after subtraction of background responses. Each symbol represents one mouse; the horizontal bar represents the median. Reference samples (mice vaccinated with pURVac DNA vaccine containing only the individual antigen linked to Ii, respectively) are not included in the statistical analysis (multiple comparison adjustment).

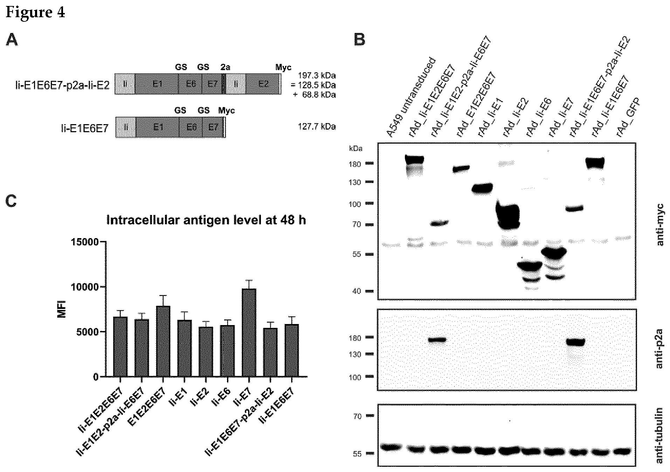

**Figure 4:** Expression analysis of rAd-shuttled MfPV3 antigen constructs. A. Schematic representation of the MfPV3 antigen variants Ii-E1E6E7-p2a-Ii-E2 and Ii-E1E6E7. MfPV3 antigens were designed as fusion proteins comprising both E1, E6 and E7 altogether, optionally fused to the MHC-II invariant chain (Ii) and E2, respectively. Theoretical molecular weight in kDa calculated from amino acid sequence. B. Western blot analysis of A549 cell lysates 48 h following transduction with rAds at an MOI of 30. Antigens were detected with anti-myc (upper panel) and anti-p2a-peptide (middle panel) antibodies. As loading control, tubulin levels were monitored using an anti-tubulin antibody (lower panel). C. Flow cytometry analysis of A549 cells 48 hours following transduction with rAds expressing the various MfPV3 antigens, at an MOI of 30. Intracellular staining was performed with anti-myc antibody. Cells were gated on non-infected cells. Depicted is the mean fluorescence intensity (MFI) of the average of 3 independent experiments. Error bars indicate standard error of the mean.

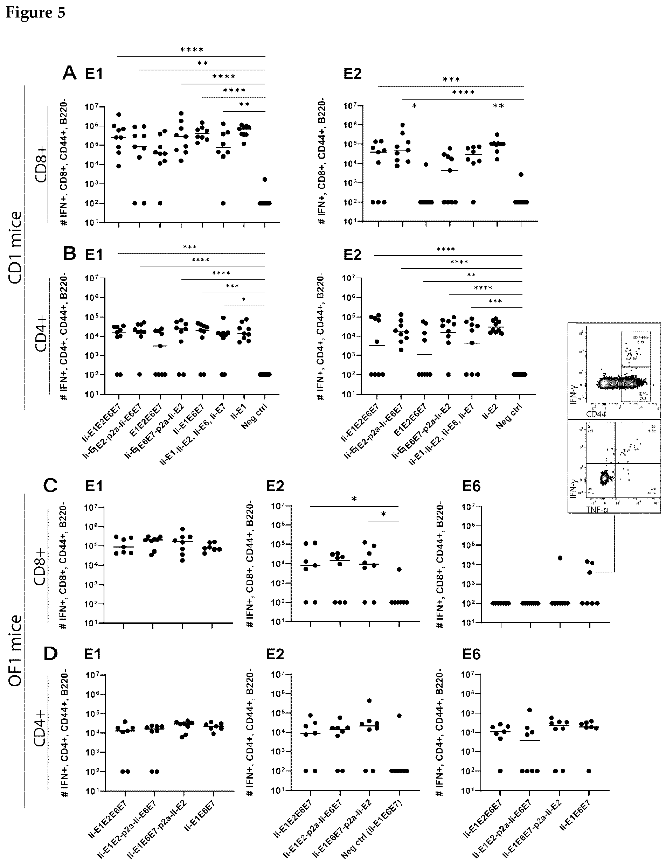

**Figure 5:** Immunization of mice with adenovirus vectors induces potent cellular immune responses. CD1 mice (A and B), or OF1 mice (C and D) were immunized with rAd vaccine ($2\times10^7$ IFU) encoding the various MfPV3 early antigens as indicated. Mice were sacrificed on day 14, spleens were harvested and CD8 and CD4 T-cell immune responses against E1, E2, E6, and E7 were measured using ICS and flow cytometry. Negative control groups consist of all mice immunized with rAd encoding antigens not covered by the peptide pools used for in vitro restimulation. Each symbol represents one mouse; the horizontal bar represents the median. Positive control samples (mice vaccinated with rAd containing only the relevant antigen linked to Ii) are not included in the statistical analysis (multiple comparison adjustment).

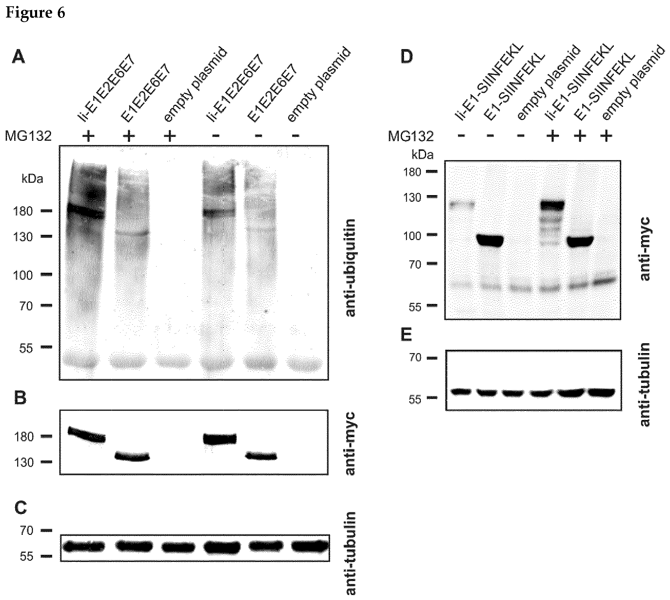

**Figure 6:** Influence of Ii on ubiquitination and degradation. A-C. pURVac Ii-E1E2E6E7, pURVac E1E2E6E7 or the empty plasmid were transfected into HEK293T cells. 24 h after transfection, cells were treated with MG132 or DMSO as control for 6 h. Myc-tagged proteins were immunoprecipitated and analyzed by western blot using an anti-ubiquitin antibody (A) and anti-myc antibody (B). Tubulin levels were monitored using an anti-tubulin antibody as loading control (C). D-E. pURVac Ii-E1-SIINFEKL, pURVac E1-SIINFEKL or empty plasmid were transfected into HEK293T cells. 24 h after transfection, cells were treated with MG132 or DMSO for 6 h. The samples were analysed by western blot using anti-myc antibody (D) and anti-tubulin antibody (E).

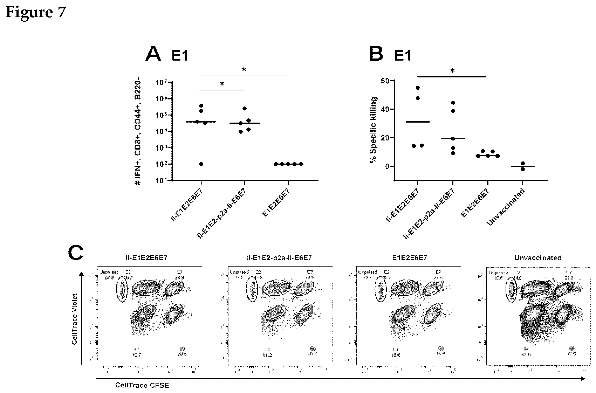

**Figure 7:** Immune responses in inbred mice and in vivo cytotoxicity Balb/C mice were immunized with rAd vectored vaccine encoding the indicated MfPV3 early antigens. 14 days post vaccination, immune responses were analyzed by ICS (A) or in vivo cytotoxicity regarding specific killing of E1-peptide pulsed cells (B). Each symbol represents one mouse. Two unvaccinated mice were included as negative controls. (C) Representative plot of in vivo cytotoxicity.

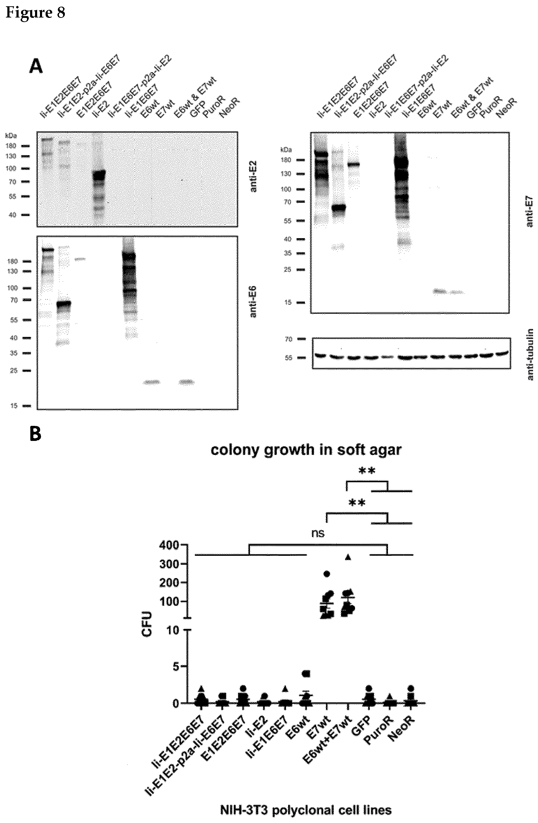

**Figure 8:** Characterization of anchorage-independent growth. A. Analysis of transgene expression of polyclonal NIH-3T3 cell lines by western blot analysis with anti-E2, anti-E6, and anti-E7 antibodies. The anti-tubulin panel confirms uniform loading. B. Anchorage-independent colony growth in soft agar transformation assay. The number of colony-forming units (CFU) in the assay is shown for each NIH-3T3 cell line. Depicted is the mean with SEM of 3 technical replicates from 3 independent experiments. Individual groups were compared using two-sided, parameterized, unpaired t-test.

| | |
|---|---|
| **Figure 9:** | Amino acid sequence alignments of E2, E6 and E7 proteins from preferred species. The alignment shows the wild-type (wt) amino acid sequence of the preferred papillomavirus species aligned with antigen sequences disclosed herein (without the "wt" qualifier), which possess the preferred modifications in accordance with the invention. The preferred modifications are highlighted with boxes. |
| **Figure 10:** | Ad19a/64 prime with MfPV3 Ii-E1E2E6E7 induced IFN-producing MfPV3 specific T-cells in NHPs after 14 days after prime, measured by ELISpot |
| **Figure 11:** | Ad19a/64 prime, MVA boost vaccination with MfPV3 Ii-E1E2E6E7 induced IFN-producing MfPV3 specific T-cells in NHPs at the day of boost (6 weeks after prime) and 9 days after boost measured by ICS and flow cytometry |
| **Figure 12:** | MfPV3 viral load in the cervix of NHPs over time. Ad19a/64 prime, MVA boost vaccination with MfPV3 Ii-E1E2E6E7 cleared the infection in all vaccinated animals, compared to spontaneous clearance in 3 out of 6 of PBS injected negative ctrl NHPs. |
| **Figure 13:** | T-cells responses after Ad19a/64 prime, rAd5 boost vaccination of CD1 mice with vaccines encoding HPV16 antigens as indicated. All constructs are immunogenic, and the preferred order of the antigens is E1 followed by E2, E6 and E7 |
| **Figure 14:** | Ad19a/64 prime, MVA boost vaccination of outbred mice with HPV16 Ii-E1E2E6E7 induced IFN-producing HPV16 specific CD8 and CD4 T-cells against all four vaccine antigens (E1, E2, E6 and E7) measured by flow cytometry 10 days after boost. This shows that this vaccine regimen is indeed immunogenic and all antigens are properly expressed and processed. |
| **Figure 15:** | Ad19a/64 prime, followed by MVA or rAd5 boost vaccination of C57BL6 mice with HPV16 Ii-E1E2E6E7. Both prime boost regimens induced IFN-producing HPV16 specific CD8 and CD4 T-cells against HPV16 E1 measured by flow cytometry 10 days after boost. This shows that rAd5 and MVA are interchangeable as boosters after an Ad19 prime vaccination, in regards of magnitude of immune responses. |
| **Figure 16:** | Therapeutic effect on early tumors. HPV16+ C3 tumor growth is reduced, and survival is improved in C57BL/6 mice receiving HPV16 Ii-E1E2E6E7 Ad19 vaccination on day 2 and Ad5 vaccination on day 20 after tumor challenge. The vaccinations also increases survival. |
| **Figure 17:** | Therapeutic effect on established tumors. HPV16+ C3 tumor growth is reduced, and survival is improved in C57BL/6 mice receiving HPV16 Ii-E1E2E6E7 Ad19 vaccination on day 10 and Ad5 vaccination on day 24 after tumor challenge. The vaccinations also increases survival (A). The control of tumor growth is enhanced by co-treatment with cisplatin (B). |

The Examples illustrate the invention.

## Example 1: Constructs of the invention and their performance

Methods

*Antigen sequences*

Parts of the antigens were synthesized at Geneart/Thermo Fisher (Regensburg, Germany). The gene optimizer algorithm was used to minimize sequence homology and adapt the sequences to human codon usage. All constructs were cloned using standard molecular biology methods. Briefly, antigens were assembled with fusion PCR, type IIs exocutter sites (Bsal-HF v2, New England Biolabs, Ipswich, USA) or NEBuilder HIFI DNA Assembly Kit (New England Biolabs, Ipswich, USA) according to manufacturer's instructions. Constructs were cloned into the different plasmid backbones using Agel-HF and Notl-HF (New England Biolabs, Ipswich, USA). Sequence correctness was verified by Sanger sequencing. Plasmids were prepared, depending on amount, with alkaline lysis or commercially available kits according to manufacturer's instructions (Plasmid Midi plus, EndoFree Plasmid Mega Kit, Qiagen, Hilden, Germany). The sequence of the antigen Ii-E1E2-p2a-Ii-E6E7 (Fig. 1) was assembled by connecting the amino acid sequence of Ii (NM_004355.3) with E1, E2, E6, and E7 of MfPV3 (EF558839.2). Following E2, the porcine teschovirus-1 2A sequence (Liu, Z. et al. Systematic comparison of 2A peptides for cloning multi-genes in a polycistronic vector. Sci. Rep. 7, 2017) was inserted, followed by a second Ii-sequence. E1 was linked to E2, and E6 to E7, via a GS-linker, respectively. Mutations were introduced into E6 and E7 to inactivate the oncogenic potential: L110Q and deletion of C-termial ETEV in E6; D24G, L71R, C95A; C297A was introduced into E2 to inactivate DNA-binding. The sequence encoding this fusion protein was optimized and adapted to human codon-usage by the GeneOptimizer-algorithm (Raab, D., Graf, M., Notka, F., Schödl, T. & Wagner, R. The GeneOptimizer Algorithm: using a sliding window approach to cope with the vast sequence space in multiparameter DNA sequence optimization. Syst. Synth. Biol. 4, 215-225, 2010), synthesized by GeneArt/ThermoFisher (Regensburg, Germany) and cloned into pDONR221. The other constructs (figure 1) were built by using standard molecular cloning techniques using the above construct as template and cloned into the desired plasmid backbones. DNA for vaccination purposes was produced using the EndoFree Plasmid Mega Kit (Qiagen, Hilden, Germany) according to the manufacturer's instructions.

*Cell lines, transfection and viral infection*

HEK293T cells and A549 cells were maintained and grown in Dulbecco's MEM (DMEM) supplemented with 10% Fetal Calf Serum (FCS) and 1% Penicillin/Streptomycin (Pen/Strep). 9E10 mycl hybridoma cells were cultivated in RPMI supplemented with 10% FCS, 1% Pen/Strep and 2 mM glutamine (Pan). All cell lines were maintained at 37°C and 5% $CO_2$ in a non-humidified incubator.

HEK293T cells were transfected using the polyethylenimide (PEI) method (Boussif, O. et al. A versatile vector for gene and oligonucleotide transfer into cells in culture and in vivo: polyethylenimine. Proc. Natl. Acad. Sci. 92, 7297 LP - 7301, 1995). For PEI transfection, $4 \times 10^5$ cells were seeded in 6-well plates one day before transfection. The cells were transfected with 2.5 $\mu$g plasmid (equimolar amounts, filled with empty vector) and 7.5 $\mu$g PEI in DMEM without any supplements. After 6 h incubation, medium was exchanged to DMEM with 10% FCS and 1% Pen/Strep.

Subconfluent A549 cells were infected with Ad19a/64 vectors at an MOI of 30 in DMEM without any supplements. 2 h post infection, medium was exchanged to DMEM with 10% FCS and 1% Pen/Strep.

*Generation of adenoviral vectors*

E1/E3 deficient adenoviral vectors of serotype Ad19a/64 were generated as previously described (Ruzsics, Z., Lemnitzer, F. & Thirion, C. Engineering Adenovirus Genome by Bacterial Artificial Chromosome (BAC) Technology BT - Adenovirus: Methods and Protocols. in (eds. Chillón, M. & Bosch, A.) 143-158 (Humana Press, 2014). doi:10.1007/978-1-62703-679-5_11) . Briefly, the Genes of Interest (GOI) -(Figure 1) were cloned into the shuttle vector pO6-19a-HCMV-MCS under control of a CMV promoter. The CMV-GOI-SV40-pA was then transferred via Flp-recombination in *E. coli* into a BAC vector (Ruzsics, Z. et al, 2014) containing the genome of a replication deficient Ad-based vector deleted in E1/E3 genes. Recombinant viral DNA was released from the purified BAC-DNA by restriction digest with PacI. The obtained linear DNA was transfected into the production cell line disclosed above for virus propagation. Recombinant viruses were released from cells via sodium deoxycholate treatment. Residual free DNA was digested by DNase I. Afterwards, vectors were purified by CsCl gradient ultracentrifugation followed by a buffer exchange to 10 mM Hepes pH 8.0, 2 mM $MgCl_2$ and 4% Sucrose via PD10 columns (GE Healthcare, Chicago, USA). Titration was performed using the RapidTiter method by detection of infected production cells via immunohistochemical staining with anti-hexon antibody (Novus, Adenovirus Antibody (8C4)). Insert integrity was confirmed by PCR amplification of the GOI in DNA extracted from the purified vectors.

*Antibodies and antibody purification*

The antibody against myc (9E10) was obtained from hybridoma cell supernatants. 9E10 mycl hybridoma cells were seeded at $5 \times 10^5$ cell per ml in RPMI supplemented with 1% FCS, 1% Pen/Strep and 2 mM glutamine. The supernatant was harvested 5 days after seeding and the antibody was purified via a HiTrap Protein G column (GE Healthcare, Chicago, USA). After washing the column with PBS, the antibody was eluted with 0.1 M glycine/HCl (pH 3.2), neutralized with 0.025 volumes of 1 M Tris/HCl (pH 9) and dialyzed against PBS.

Other antibodies used were: mouse anti-p2a peptide (3H4, 1:2000, Merck, Darmstadt, Germany), mouse anti-tubulin (DM1$\alpha$, 1:1000, Santa Cruz, Heidelberg, Germany), mouse anti-ubiquitin-Biotin (eBioP4D1, 1:1000, Invitrogen, Carlsbad, USA), goat anti-mouse-HRP (115-036-003, 1:5000, Jackson, West Grove, USA), goat anti-rabbit-HRP (P0448, 1:2000, Dako, Santa Clara, USA), Streptavidin-HRP (11089153001, 1:5000, Roche, Basel, Swiss), rat anti-mouse-PE (A85-1, 1:100, BD, Franklin Lakes, USA).

*Western blot analysis*

Western blot analysis was performed as previously described (Kiener, R. et al. Vaccine vectors based on Adenovirus 19a/64 exhibit broad cellular tropism and potently restimulate HCMV-specific T cell responses ex vivo. Sci. Rep. 8, 1474, 2018). Briefly, cells of interest were lysed in TDLB buffer (50 mM Tris, pH 8.0, 150 mM NaCl, 0.1% SDS, 1% Nonident P-40, 0.5% sodium deoxycholate) supplemented with protease inhibitors (Complete Mini, Roche, Basel, Swiss). Total protein concentration of the supernatants was measured by the Bradford method (Protein Assay, BioRad, Feldkirchen, Germany). The proteins were separated on SDS-PAGE under reducing conditions and blotted on a nitrocellulose membrane for western blot analysis. Targets were probed with primary and secondary antibodies as listed above. HRP-labeled secondary antibodies and enhanced chemiluminescence substrate or Femto ECL (Thermo Fisher, Waltham, USA) were used for detection in a Chemilux Pro device (Intas, Göttingen, Germany). For loading control, the membrane was reprobed with an antibody against tubulin.

*Flow cytometry analysis of cell lines*

Intracellular staining of antigens was performed using standard methods (Kiener et al., 2018). Cells were fixed and permeabilized with Cytofix/Cytoperm-Buffer (4% PFA, 1% saponine, in PBS). All washing steps were done with Perm/Wash-Buffer (PBS containing 0.1% saponine). The cells were stained with anti-myc antibody (5 $\mu$g/ml, diluted in Perm/Wash-Buffer) and rat anti-mouse-PE (1:100 diluted in Perm/Wash-Buffer) each for 30 min.
The flow cytometry assay was performed using an Attune NxT device (Thermo Fisher, Waltham, USA) with a 488 nm excitation and a 574/26 nm emission filter. Cells were gated on stained, mock-transfected cells. Evaluation of data was performed using Attune NxT software.

*Analysis of ubiquitination*

To analyse ubiquitinylated proteins, 24 h post transfection, cells were treated with 10 $\mu$M MG132 proteasome inhibitor for 6 h. Afterwards, cells were harvested in PBS and washed twice. For inactivation of deubiquitination enzymes, 20 mM N-ethylmaleimide from a freshly prepared stock solution were added to the TDLB lysis buffer. Lysates were generated as described above. Before immunoprecipitation, Protein G dynabeads (Thermo Fisher, Waltham, USA) were loaded with 10 $\mu$g of pulldown antibody. Using these beads, target protein was immunoprecipitated out of 500 $\mu$g cell lysate over night at 4°C under slow rotation. After washing the beads four times with PBS, SDS-PAGE buffer was added to the beads before heating at 95°C for 10 min. The samples were used for western blot analyses as described above.

*Animals and immunizations*

Balb/C and CD1 female mice were obtained from Envigo (Horst, The Netherlands) and OF1 mice from Charles River (France). All animals were housed at the Panum Institute, University of Copenhagen. All experiments were initiated after allowing the mice to acclimatize for at least 1 wk. Experiments were approved by the National Animal Experiments Inspectorate (Dyreforsøgstilsynet, license no. 2016-15-0201-01131) and performed according to national guidelines.
DNA immunizations were performed intradermal (i.d.) with 0.5 g DNA coated onto 1.6 $\mu$m gold microcarriers (BioRad, Feldkirchen, Germany) using the Helios Genegun System (BioRad, Feldkirchen, Germany). The mice received four DNA immunizations at intervals of one week each. One group received a mixture of four plasmids, 0.5 $\mu$g each, into one site. Immunizations with adenoviral vectors were performed intramuscular (i.m.) with $2 \times 10^7$ IFU Ad19a/64 diluted in 50 $\mu$L PBS. Mice were anesthetized with isofluorane before Ad19a/64 injections. One group received a mixture of four Ad19a/64, $2 \times 10^7$ IFU each, into one site.

*Flow cytometry of splenocytes*

Single cell suspensions of splenocytes were obtained by organ harvest in HANKs followed by straining through 70 $\mu$m cell strainers. Cells were incubated for 5 hours in 3 $\mu$M monensin with or without 1 $\mu$g/mL of relevant peptides. The cells were stained against surface markers: APC-Cy7 or BV421 CD8 (53-6.7, 1:200, BioLegend, San Diego, USA), PE-Cy7 CD4 (RM4-5, 1:800, BD), FITC CD44 (IM7, 1:100, BioLegend, San Diego, USA) and PerCP-Cy5.5 B220 (RA3-6B2, 1:200, BioLegend, San Diego, USA). After surface staining, cells were fixed in 1% PFA, permeabilized in 0.5% saponine, and stained intracellularly using APC IFN-$\gamma$ (XMG1.2, 1:100, BioLegend, San Diego, USA) and PE TNF-$\alpha$ (MP6-XT22, 1:100, BioLegend, San Diego, USA) antibodies.
The peptides used were 16-mers overlapping by 11 amino acids covering the entire Ii-E1E2E6E7 antigen. The peptides were pooled in 5 separate pools containing Ii, E1, E2, E6 and E7 peptides respectively. Peptides were obtained from KareBay, Town, China.
Flow cytometry was performed on the Fortessa 3 (BD Biosciences, Franklin Lakes, USA) flow cytometer and data analysis was performed using FlowJo V10 software. Epitope-specific CD8$^+$ T-cell responses were measured as B220-, CD8$^+$ or CD4$^+$, CD44$^+$, IFN-$\gamma^+$ cells and are presented in total number of cells per organ. The quality of the IFN-

$\gamma^+$ responses were evaluated by MFI of IFN-$\gamma$ and fraction of double positive cells (expressing both IFN-$\gamma$ and TNF) in the IFN-$\gamma^+$ CD8$^+$ or CD4$^+$ populations.

*In vivo cytotoxicity*

The assay was performed similarly to what was previously described (Nielsen, K. N., Steffensen, M. A., Christensen, J. P. & Thomsen, A. R. Priming of CD8 T cells by adenoviral vectors is critically dependent on B7 and dendritic cells but only partially dependent on CD28 ligation on CD8 T cells. J. Immunol. 193, 1223-1232, 2014). Briefly, splenocytes from naive Balb/C mice were incubated with MfPV3 E1, E2, E6 or E7 peptide pools or no peptide for 30 minutes at 37°C, 5% $CO_2$, 2.5 $\mu$g of each peptide/mL, and subsequently stained with combinations of 0.4 or 5 $\mu$MCellTrace CFSE and 0.2 and 2.5 $\mu$MCellTraceViolet (CTV; ThermoFisher, Waltham, USA) for 10 minutes at 37°C, 5% $CO_2$. Pulsed and stained splenocytes were mixed at a 1:1:1:1:1 ratio, and a total of 2.5×10$^7$ cells were injected intravenously into Ad19a/64-vaccinated recipient Balb/C mice. 5 hours later, spleens were harvested, and target cells were identified on the Fortessa 3 (BD Biosciences, Franklin Lakes, USA) flow cytometer by CFSE/CTV staining. The percentage of killing was calculated using the following equation:

$$\% \, targeted \, killing = 100 - \left( \frac{\frac{\% \, peptide \, pulsed \, cells \, in \, vaccinated \, mice}{\% \, non-peptide \, pulsed \, cells \, in \, vaccinated \, mice}}{\frac{\% \, peptide \, pulsed \, cells \, in \, non-vaccinated \, mice}{\% \, non-peptide \, pulsed \, cells \, in \, non-vaccinated \, mice}} \right) * 100$$

*Analysis of E1 (SIINFEKL) presentation on MHC-I*

HEK293T cells were transfected with 2.5-3.5 $\mu$g DNA using PEI transfection. 48 hours post transfection, cells were stained with PE anti-H2KB-SIINFEKL (25-D1.16, 1:160, Invitrogen, Carlsbad, USA) and presence of SIINFEKL-H2Kb presentation on cell surfaces was detected on the LSRII or Fortessa 3 (BD Biosciences, Franklin Lakes, USA) flow cytometers, as a proxy for E1 presentation. All samples were run in biological 6-plicates, and the experiment was repeated at least two times.

*Graphical representation and statistical analysis of ex vivo immunogenicity data*

Non-stimulated samples were used as background controls, and their response values have been subtracted from the peptide-stimulated samples of the corresponding animal before performing statistical analysis and graphical presentation.

Statistical analysis was done using GraphPad Prism 8 software (GraphPad Software, San Diego, USA). Values with background subtracted were used to compare the individual groups using the Mann-Whitney test with Bonferroni correction for multiple comparisons. Positive control groups were not included in the multiple comparison analyses.

In order to aid visual presentation of the results, we applied a threshold for responses based on the average number of B220-, CD8$^+$, CD44$^+$, IFN-$\gamma^+$ counts for unstimulated background samples. All samples with less counts than the average + 2 × SD of the background samples, are regarded as non-responding and therefore they have manually been adjusted to a value of 100 for the graphical presentation. All statistical analyses were done on non-adjusted values.

The analysis of fraction of double positive (% TNF$^+$ IFN-$\gamma^+$ out of all IFN-$\gamma^+$) and MFI of IFN-y$^+$ was only performed on responders (IFN-$\gamma^+$ count > avg + 2 × SD of unstimulated samples). Each symbol represents one mouse. Bars indicate median. Significance levels are marked by * (p<0.05), ** (p<0.01), *** (p<0.005).

Results

*Design of the antigens*

With the aim of generating a therapeutic vaccine capable of eliminating pre-existing papillomavirus infections in non-human primates, antigen constructs comprising E1, E2, E6, and E7 of MfPV3 linked to the intrinsic T-cell adjuvant Ii were designed. Eight different configurations were conceived (figure 1) to select the one eliciting the most potent and broadest response for further development. While in the first composite antigen the order was Ii-E1E2E6E7, the order of E1/E2 and E6/E7 was reversed in Ii_E6E7E1E2 (i) to test the influence of the antigen order, and (ii) to determine the impact of positioning relative to Ii on the level of transgene expression. In both cases, all proteins were expressed as artificial fusion polypeptides, whereas the individual proteins were separated by a short and flexible GS linker to promote the stability and accumulation of the fusion proteins (Chen, X., Zaro, J. L. & Shen, W.-C. Fusion protein linkers: property, design and functionality. Adv. Drug Deliv. Rev. 65, 1357-1369, 2013) and Ii was fused to the N-terminus as a T cell adjuvant (Holst, P. J. et al. MHC Class II-Associated Invariant Chain Linkage of Antigen Dramatically Improves Cell-Mediated Immunity Induced by Adenovirus Vaccines. J. Immunol. 180, 3339-3346, 2008). As a reference to assess the T cell adjuvant effects of Ii, the variant E1E2E6E7 without Ii was designed. To investigate if the Ii adjuvant effect was more pronounced when fused to shorter antigens, E1E2 were separated from E6E7 by a p2a peptide in Ii-E1E2-p2a-Ii-E6E7 (Kim, J. H. et al. High cleavage efficiency of a 2A peptide derived from porcine teschovirus-1 in human cell lines, zebrafish and mice. PLoS One 6, 2011). The nucleotide sequence homology between the two invariant chains was minimized by using divergent codons to hinder homologous recombination. Additionally, each of the four viral proteins was generated on its own as Ii-fusion to serve as benchmark and reference. Because of the lack of antibodies against MfPV3 early proteins, all constructs were modified to contain a C-terminal myc-tag.

Since E6 and E7 are highly potent oncoproteins, only transformation-defective variants should be used in the setting of a vaccine to ensure safety. Based on the homology to HPV16, the oncoprotein E6 was inactivated by introducing a L110Q substitution to prevent binding of E6 to E6BP and therefore binding and degradation of tumor suppressor p53 (Liu, Y. et al. Multiple Functions of Human Papillomavirus Type 16 E6 Contribute to the Immortalization of Mammary Epithelial Cells. 73, 7297-7307, 1999). Additionally, the C-terminal PDZ-domain was deleted (ΔETEV) to abolish binding of telomerase and other LxxLL proteins (Wieking, B. G. et al. A Non-oncogenic HPV 16 E6/E7 Vaccine Enhances Treatment of HPV Expressing Tumors. Cancer Gene Ther. 19, 667-674, 2013). E7 was inactivated by introducing the substitutions C24G, L71R, and C95A, which inactivate the central LxCxE motif to reduce transforming activity as well as pRB binding, reduce Mi2β binding, and inhibit dimerization, respectively (Wieking, 2013; Edmonds, C. & Vousden, K. H. A Point Mutational Analysis of Human Papillomavirus Type 16 E7 Protein. 63, 2650-2656,1989). The conserved DNA binding domain of E2 was modified by C297A to generate polyproteins with an even number of cysteins and reduce DNA binding.

For initial biochemical and immunological characterization, the constructs were cloned into pURVac, a derivative of a DNA vaccine vector with a proven track record in various NHP and clinical trials (Asbach, B. et al. Priming with a Potent HIV-1 DNA Vaccine Frames the Quality of Immune Responses prior to a Poxvirus and Protein Boost. J. Virol. 93,2019; Sarwar, U. N. et al. Safety and immunogenicity of DNA vaccines encoding Ebolavirus and Marburgvirus wild-type glycoproteins in a phase I clinical trial. J. Infect. Dis. 211, 549-557, 2015; Joseph, S. et al. A Comparative Phase I Study of Combination, Homologous Subtype-C DNA, MVA, and Env gp140 Protein/Adjuvant HIV Vaccines in Two Immunization Regimes. Front. Immunol. 8, 149, 2017; Pantaleo, G. et al. Safety and immunogenicity of a multivalent HIV vaccine comprising envelope protein with either DNA or NYVAC vectors (HVTN 096): a phase 1b, double-blind, placebo-controlled trial. Lancet HIV 6, e737-e749, 2019) where antigens are under the control of a human cytomegalovirus (CMV) promoter in combination with a human T-cell leukemia virus -1 (HTLV-1) regulatory element (Barouch, D. H. et al. A human T-cell leukemia virus type 1 regulatory element enhances the immunogenicity of human immunodeficiency virus type 1 DNA vaccines in mice and nonhuman primates. J. Virol. 79, 8828-8834, 2005) and a bovine growth hormone poly-A terminator.

*Biochemical and cell biological characterization of MfPV3 antigens*

Initially, expression properties of the antigen constructs were evaluated. All antigens were expressed in HEK293T cells with bands of the expected sizes being detected by western blot analysis using an anti-myc antibody (figure 2A; figure 1). A higher molecular weight band for a non-separated Ii-E1E2-p2a-Ii-E6E7 could neither be detected in the anti-myc western-blot detecting the C-terminal fragment, nor in the anti-p2a western blot where the N-terminal fragment is detected. This provides evidence that the polyprotein was completely separated at the p2a site. In some cases, especially for proteins with high signal intensities, bands of higher or lower molecular weight as compared to the calculated values could be observed. Based on their sizes, the bands of higher molecular weight may resemble incompletely reduced proteins, which is not completely surprising as Ii seems to form cysteine linked trimers (Fougeroux, C., Turner, L., Bojesen, A. M., Lavstsen, T. & Holst, P. J. Modified MHC Class II-Associated Invariant Chain Induces Increased Antibody Responses against Plasmodium falciparum Antigens after Adenoviral Vaccination . J. Immunol. 202, 2320-2331, 2019). Bands of lower molecular weight presumably resemble N-terminal degradation products that are most likely cleaved within Ii as based on the fragment sizes. Partial degradation is expected as Ii transports some of its linked cargo to endosomal compartments where proteolytic degradation occurs.

Since proteins of different sizes exhibit different blotting conditions, the western blot analysis could not be assumed to be quantitative, so signal intensities may not reflect the real expression levels. Therefore, protein levels were quantified by flow cytometry after intracellular staining with the anti-myc-antibody (figure 2B). Expression levels of the different MfPV3 antigens differed only marginally, except for Ii-E7, which exhibited two- to threefold higher MFI values compared to all other antigens.

*MHC class I-restricted SIINFEKL epitope is processed from E1 fusion protein and abundantly presented on MHC-I molecules in vitro*

It has been reported that bovine papillomavirus type 1 E1 is itself an unstable protein which is ubiquitinylated and rapidly degraded in papillomavirus-infected cells (Malcles, M.-H., Cueille, N., Mechali, F., Coux, O. & Bonne-Andrea, C. Regulation of bovine papillomavirus replicative helicase e1 by the ubiquitin-proteasome pathway. J. Virol. 76, 11350-11358, 2002; Mechali, F., Hsu, C.-Y., Castro, A., Lorca, T. & Bonne-Andrea, C. Bovine papillomavirus replicative helicase E1 is a target of the ubiquitin ligase APC. J. Virol. 78, 2615-2619 (2004). The constitutive degradation might explain why we have been unable to find any studies reporting the detection of E1 expression at the protein level in HPV infected cells and tissues, and why E1 is poorly immunogenic with respect to antibodies (Ewaisha, R., Panicker, G., Maranian, P., Unger, E. R. & Anderson, K. S. Serum Immune Profiling for Early Detection of Cervical Disease. Theranostics 7, 3814-3823, 2017). However, it should be confirmed that, despite being poorly immunogenic in natural HPV infections, E1-derived peptides are presented on MHC-I on infected cells, a prerequisite for being targetable by therapeutic vaccination. To detect presentation of E1-derived peptides, we designed a DNA construct encoding MfPV3 E1 in sequence with the minimal H2-kb-restricted OVA epitope SIINFEKL (Dersh, D., Yewdell, J. W. & Wei, J. A SIINFEKL-Based System to Measure MHC Class I Antigen Presentation Efficiency and Kinetics. Methods Mol. Biol. 1988, 109-122 (2019). Following transfection of the E1-SIINFEKL construct, transgene presentation on MHC-I was assessed using an H2-Kb-SIINFEKL specific antibody (figure 2C). Processed and presented SIINFEKL-peptide could be detected on MHC-I on cell surfaces, both when linked to Ii and when delivered alone. This supports the choice of E1 as a relevant antigen to include in the therapeutic vaccine, as E1-derived peptides can be expected to be presented on the surface of infected cells.

*DNA vaccination of antigen constructs induces CD4+ and CD8+ T cell responses against MfPV3 early antigens*

Intradermal (i.d.) DNA immunization of outbred CD1 mice confirmed that all pURVac DNA vaccines encoding E1 and/or E2 induced E1- and E2-specific IFN-$\gamma^+$ CD8$^+$ and CD4$^+$ T cells measured by ICS and flow cytometry analysis (figure 3. More detailed, all DNA vaccine candidates had provided an E1 response on par with the positive control, whereas there was a trend to higher responses for the Ii-E2 positive control. No vaccines induced responses against E6 or E7 in this outbred strain of mice, including the positive controls for these two antigens. Importantly, all vaccines encoding all four antigens as a polyprotein generated responses comparable to those induced by a mix of four vaccines encoding the antigens individually (Ii-E1, Ii-E2, Ii-E6, Ii-E7). This suggests that overall no immunogenicity was lost by delivering the MfPV3 early antigens as polyproteins from a single pURVac DNA vaccine. Altogether, the four different polyprotein encoding DNA vaccines induced T cell responses, which showed no statistical differences regarding the frequencies of E1- and E2-specific IFN-$\gamma^+$ CD8$^+$ and CD4$^+$ T cells. This is the case regardless (i) of whether Ii is fused to E1E2E6E7, (ii) of the order of the early antigens in the polyprotein, and (iii) of the use of a p2a site to separate Ii-E1E2 from Ii-E6E7. The quality of the T-cell responses was comparable across all vaccines, assessed by MFI of IFN-$\gamma$ and TNF production of IFN-$\gamma^+$ T-cells. As expected, Ii gave rise to CD8 responses in most mice, and to CD4 in some mice, as the human Ii is allogenic in mice. The vaccine with two p2a-separated Ii-linked antigen sequences (Ii-E1E2-p2a-Ii-E6E7) and Ii-E6E7E1E2 appeared to elicit slightly better CD4 responses against E1 shown by the significant response and significant difference to the negative control group (figure 3A).

*Characterization of adenoviral vectors*

To increase the cellular immune response against the antigens, viral vectors were subsequently used for antigen delivery. Adenoviral vectors from serotype 19a/64 (rAd) had been shown to be a suitable vector for the delivery of MfPV3 antigens that were capable of inducing high CD8$^+$ T cell response in cynomolgus macaques (Ragonnaud, E. et al. Replication deficient human adenovirus vector serotype 19a/64: Immunogenicity in mice and female cynomolgus macaques. Vaccine 36, 6212-6222, 2018). A refined panel of rAd vectors was generated comprising a modified set of recombinant MfPV3 antigens. Ii-E6E7E1E2 was excluded because it was not superior to the other polyproteins. The trend toward higher responses against E2 when administered alone led us to speculate whether it could be advantageous to have E2 individually linked to Ii and expressed as separate domain. Hence, a vaccine encoding an N-terminal Ii linked to E1, E6 and E7 (Ii-E1E6E7) without E2 as reference, and a version in which the Ii-E1E6E7 antigen was extended by an Ii-E2 fusion via a self-separating p2a peptide (Ii-E1E6E7-p2a-Ii-E2) was designed. In order to confirm proper expression of the encoded antigens, western blot analysis was performed with adenovirally transduced A549 cells. All vectors readily expressed the antigens with bands resembling the respective fusion proteins being detected at the expected positions (figure 4A, figure 1 and 2). As for Ii-E1E2-p2a-Ii-E6E7, no higher molecular weight fragment could be detected for Ii-E1E6E7-p2a-Ii-E2, revealing that the complete separation at the p2a site also worked in the context of adenoviral-vector-delivered antigens. Quantitative comparison of the adenovirally delivered antigens in A549 cells using flow cytometry confirmed similar expression levels of the different MfPV3 antigen constructs with the exception of higher expression of the Ii-E7 fusion protein (figure 4B. The overall picture was quite similar to the expression observed after delivery of pURVac DNA vaccine vectors in HEK293T cells.

*Adenoviral delivery induces potent cellular immune responses against HPV early antigens in outbred mice*

Intramuscular immunization of outbred CD1 mice confirmed that the Ad19a/64 -formulation of the vaccines induced both CD8$^+$ and CD4$^+$ T cell responses against E1 and E2 antigens (figure 5A and B). Notably, vaccines including Ii showed a trend towards higher magnitude responses than the vaccine not encoding Ii (figure 5 A and B: E1E2E6E7 compared to other vaccines). A tendency of Ii-mediated enhancement of responses was observed for both CD4 and CD8 T cell responses for some vaccine configurations, but the difference was only significant for E2-specific CD8 responses induced by Ii-E1E2-p2a-Ii-E6E7 compared to E1E2E6E7 (figure 5B, upper panel). This underlines the ability of Ii to boost T cell responses in the context of adenoviral vaccine delivery. As the Ii-E1E2E6E7 antigen additionally showed a tendency towards higher E1 responses than the Ii-E1E2-p2a-Ii-E6E7 antigen, follow-on experiments were performed that, however, failed to demonstrate a consistent difference in antigen immunogenicity hierarchy between the constructs (data not shown). No difference in the quality of the T cell responses was detected, assessed by the fraction of double-positive T cells capable of secreting both IFN-γ and TNF and by the MFI values of IFN-γ.

Besides vigorous T-cell responses against E1 and E2 (figure 5C and D), we did indeed see solid CD4$^+$ T-cell responses against E6 (figure 5D). A few mice also had CD8$^+$ T-cells reacting towards E6, confirming processing and immunogenicity of our vaccine constructs. As only a few mice had CD8$^+$ T-cell responses and these responses were of low magnitude, it could be questioned whether these were true responses or merely due to high background. A representative mouse depicted in figure 5C, shows a CD44$^+$IFN-γ$^+$ population behaving like a true response. Furthermore, the majority of the IFN-γ$^+$ cells were producing TNF as well, confirming the activation phenotype.

Overall, the Ad19a/64 delivery of the vaccines efficiently induced T-cells against all the four MfPV3 polyprotein antigens tested, and the immunogenicity of the antigens was enhanced by inclusion of Ii.

*Ii-fusion enhanced ubiquitination and proteasomal degradation*

The mechanistic basis of the T cell adjuvant effect of Ii has not been fully clarified yet (Fougeroux et al, 2019), but one of the suggested mechanisms of action is ubiquitination of Ii leading to proteasomal degradation of the linked antigen and thereby enhanced MHC-I presentation (Esposito, I. et al. MHC class II invariant chain-adjuvanted viral vectored vaccines enhances T cell responses in humans. Sci. Transl. Med. 12, (2020). To investigate if this mechanism could also account for the enhanced CD8$^+$ T-cell responses against the Ii-linked MfPV3 antigens, Ii-E1E2E6E7- and E1E2E6E7-transfected cells were cultivated in absence or presence of the proteasome inhibitor MG132. Anti-ubiquitin western blot analysis of the immunoprecipitated myc-tagged polypeptides revealed higher ubiquitin-depending signal intensity for Ii-E1E2E6E7 compared to E1E2E6E7 without Ii (figure 6A). This effect was even more pronounced when MG132 was present. Myc-specific signals confirmed the fidelity of the immunoprecipitation (IP) procedure, and analysis of the IP supernatants by an anti-tubulin western blot revealed that comparable amounts of cell lysate were used in the IP procedure (figure 6B and C). A higher level of ubiquitination is commonly associated with faster degradation. This could exemplarily be demonstrated by transiently expressing Ii-E1 and E1, both fused with the C-terminal SIINFEKL peptide, in absence or presence of MG132, respectively (figure 6D and E). Without MG132, Ii-E1-SIINFEKL was hardly detectable whereas a prominent signal could be visualized when MG132 was added. Lower molecular weight signals are indicative of degradation products. This effect was not observed for E1 without Ii, which suggested that Ii induced an accelerated proteasomal degradation.

*Adenoviral delivery induces specific killing of target cells in vivo*

To assure that the cellular responses induced by the rAd-delivered antigens had cytotoxic capacity, we immunized inbred Balb/C mice, and 14 days later challenged these mice with peptide-pulsed pre-stained syngeneic target cells. The vaccines induced CD8$^+$ responses against E1 (figure 7A), but neither against the other antigens in Balb/C mice (Data not shown), whereas CD4 responses were raised against E1, E2 and E6. Ii again proved its relevance as an adjuvant, as the vaccine without Ii did not give rise to any detectable E1 specific CD8$^+$ responses in this inbred mouse model (figure 7A). The *in vivo* cytotoxicity assay showed specific killing of E1 labelled cells (figure 7B and C), and the hierarchy of specific killing capability corresponds to the number of IFN-γ$^+$ CD8$^+$ E1 specific T cells. We can conclude that the designed rAd vaccines induce potent and cytotoxic responses, which supports our hypothesis that this vaccine design will be capable of removing existing MfPV3 infections.

**Example 2: Assessment of oncogenic properties of E6 and E7 as modified in accordance with the invention**

Material and Methods

*Cell lines*

NIH-3T3 cells and HEK293T cells were cultivated at 37°C with 5% $CO_2$ in DMEM supplemented with 10% fetal calf serum (FCS) and 1% Pen/Strep (PS). The NIH-3T3 cells were maintained between 10% and 70% confluence to prevent maturation and differentiation.

*Production of VSV-G-pseudotyped lentiviral vectors*

The HPV16 antigens and HPV16 E6wt were cloned into pLV-MCS-IRES-Puro, HPV16 E7wt and GFP were cloned into pLV-MCS-IRES-Neo, respectively, by using AgeI-HF and NotI-HF (NEB, Ipswich, USA) restriction enzymes. Both pLV vectors contain a CMV promoter followed by an MCS, an internal ribosome entry site (IRES) and antibiotic resistance gene against either puromycin or neomycin.

$3.9 \times 10^6$ HEK293T cells were seeded in T75 flasks. 24 h post seeding, the medium was changed to DMEM without additives and the cells were transfected with 3.75 μg of pLV containing the antigen and 3.75 μg of lentivirus packaging mix (1:1:1; pMDL-Gag:pVSV-G:pREV) using the PEI method (Boussif, O., Lezoualc'h, F., Zanta, M. A., Mergny, M. D., Scherman, D., Demeneix, B., & Behr, J. P. (1995). A versatile vector for gene and oligonucleotide transfer into cells in culture and in vivo: polyethylenimine. Proceedings of the National Academy of Sciences, 92(16), 7297 LP - 7301. https://doi.org/10.1073/pnas.92.16.7297) in a final volume of 9 ml. 5 h post transfection, 4 ml DMEM containing 10% FCS and 1% PS were added to the flasks. The lentiviral vectors were harvested 48 h post transfection by centrifugation at 1000×g for 5 min, the supernatants were filtered through 0.45 μm syringe filters and stored at -80°C in aliquots.

*Transduction of NIH-3T3 cells and antibiotic selection*

$1 \times 10^5$ NIH-3T3 cells were seeded in T25 flasks. 24h post seeding, the medium was aspirated, and the cells were transduced with 2 ml of each supernatant containing the respective lentiviral vector in presence of 10 μg/ml polybrene (TR-1003-G, Sigma Aldrich, St. Louis, USA). After 6 h, 4 ml DMEM containing 10% FCS and 1% PS were added. 72 hours later, transduced cells were selected via 1 mg/ml G418 (CP11.3, Roth, Karlsruhe, Germany) and/or 2 μg/ml puromycin (ant-pr-1, InVivoGen, San Diego, USA), depending on the lentiviral vector used for transduction. The cells were under antibiotic selection for at least 2 weeks, until all untransduced cells had died.

*Antibodies*

The following antibodies were used: anti-E2 (TVG-271, 1:200, Santa Cruz Biotechnology, Heidelberg, Germany), anti-E6 (GTX132686, 1:2000, Biozol, Eching, Germany), anti-E7 (NM2, 1:200, Santa Cruz Biotechnology, Heidelberg, Germany), anti-tubulin (DM1$\alpha$, 1:1000, Santa Cruz Biotechnology, Heidelberg, Germany), goat anti-mouse-HRP (115-036-003, 1:5000, Jackson, West Grove, USA) and goat anti-rabbit-HRP (P0448, 1:2000, Dako, Santa Clara, USA).

*Western blot analysis*

Western blot analysis was performed as previously described (Kiener, R., Fleischmann, M., Schwegler, C., Ruzsics, Z., Thirion, C., Schrödel, S., Asbach, B., & Wagner, R. (2018). Vaccine vectors based on Adenovirus 19a/64 exhibit broad cellular tropism and potently restimulate HCMV-specific T cell responses ex vivo. Scientific Reports, 8(1), 1474. https://doi.org/10.1038/s41598-018-19874-1). Briefly, cells of interest were lysed in TDLB buffer (50 mM Tris, pH 8.0, 150 mM NaCl, 0.1% SDS, 1% Nonidet P-40, 0.5% sodium deoxycholate) supplemented with protease inhibitors (Complete Mini, Roche, Basel, Switzerland). Total protein concentration of the supernatants was measured by the Bradford method (Protein Assay, BioRad, Feldkirchen, Germany). The proteins were separated on SDS-PAGE under reducing conditions and blotted on a nitrocellulose membrane for western blot analysis. Targets were probed with primary and secondary antibodies as listed above. HRP-labeled secondary antibodies and enhanced chemiluminescence substrate or Femto ECL (Thermo Fisher, Waltham, USA) were used for detection in a Chemilux Pro device (Intas, Göttingen, Germany). For loading control, the membrane was stripped (stripping buffer (62.5 mM Tris, 2% SDS, 0.1 M β-Mercaptoethanol, pH 6.6) for 30 min at 50°C) and reprobed with an antibody against tubulin.

*Soft agar transformation assay*

The soft agar transformation assay was performed as described previously (Borowicz, S., Van Scoyk, M., Avasarala, S., Karuppusamy Rathinam, M. K., Tauler, J., Bikkavilli, R. K., & Winn, R. A. (2014). The Soft Agar Colony Formation Assay. JoVE, 92, e51998. https://doi.org/doi:10.3791/51998) with minor modifications as follows. 6-well plates were filled with 2 ml DMEM (without phenol red, low glucose) (D2902, Sigma Aldrich, St. Louis, USA) containing 10% FCS, 1% PS, 3.7 g/l NaHCO$_3$, 3.8 g/l D-glucose, 0.5% low melt agarose (6351.2, Roth, Karlsruhe, Germany), 1 mg/ml G418 and/or 2 $\mu$g/ml puromycin, respectively, and incubated at room temperature until the medium had solidified. $5\times10^3$ of the lentivirally transduced polyclonal NIH-3T3 cells were seeded in 37°C-prewarmed DMEM containing 10% FCS, 1% PS, 3.7 g/l NaHCO$_3$, 3.8 g/l D-glucose, 0.35% low melt agarose, 1 mg/ml G418 and/or 2 $\mu$g/ml puromycin, respectively, onto the pretreated 6-well plates and incubated at room temperature, until the medium had solidified. The plates were incubated at 37°C and 5% CO$_2$ for 21 days. Within this period, the cells were fed twice a week with 200 $\mu$l of DMEM, supplemented with 10% FCS and 1% PS. After 21 days, each well was fixed with 100 $\mu$l 4% PFA and stained with 0.005% crystal violet in 10% methanol (v/v) for 1 h at room temperature. Pictures were taken on an alphaimager HP (Proteinsimple, San Jose, USA) at an aperture of 5.6, and focus of 2.88. The colonies were counted with ImageJ (Schindelin, J., Arganda-Carreras, I., Frise, E., Kaynig, V., Longair, M., Pietzsch, T., Preibisch, S., Rueden, C., Saalfeld, S., Schmid, B., Tinevez, J.-Y., White, D. J., Hartenstein, V., Eliceiri, K., Tomancak, P., & Cardona, A. (2012). Fiji: an open-source platform for biological-image analysis. Nature Methods, 9(7), 676-682. https://doi.org/10.1038/nmeth.2019). Statistical analysis was done using GraphPad Prism 8 software (GraphPad Software, San Diego, USA). Individual groups were compared using two-sided, parameterized, unpaired t-test. Each symbol represents one replicate, bars indicate mean with standard error of the mean (SEM). Significance levels are marked by * (p<0.05), ** (p<0.005), ns (not significant).

Results

*Antigen-expression did not cause anchorage-independent growth*

Anchorage-independent growth is a hallmark of carcinogenesis, since transformed cells are able to grow independently of attachment to a solid phase (Borowicz et al., 2014). One of the most stringent tests for transformation is the soft agar transformation assay, which characterizes solid-phase-independent growth in soft agar. For this, NIH-3T3 cells were transduced with VSV-G-pseudotyped lentiviral vectors encoding the following HPV16 antigens: Ii-E1E2E6E7, Ii-E1E2-p2a-Ii-E6E7, E1E2E6E7, Ii-E2, Ii-E1E6E7-p2a-Ii-E2, Ii-E1E6E7, E6wt, and E7wt, respectively (figure 1A). For control purposes, NIH-3T3 cells were transduced with VSV-G-pseudotyped lentiviral vectors encoding GFP and lentiviral vectors lacking a transgene, respectively. Additionally, one polyclonal NIH-3T3 cell line was generated by transduction with both, HPV16-E6wt- and HPV16-E7wt-encoding lentiviral vectors.

After antibiotic selection, transgene expression of polyclonal NIH-3T3 cell lines was verified by western blot analysis. For all cell lines, the expected bands at the correct sizes were detected, except for Ii-E1E6E7-p2a-Ii-E2 (figure 8A). As generation of this cell line failed in 3 attempts, Ii-E1E6E7-p2a-Ii-E2 was excluded from further analysis. $5\times10^3$ cells were seeded in soft agar as described in the methods section. 3 weeks after seeding, the plates were stained, and colonies were counted with ImageJ. There was no difference in the number of colonies as compared to the negative controls (GFP- and empty-vector-transduced NIH-3T3 cells), except for E7wt- and E6wt+E7wt-transduced cells (figure 8B) that readily formed high numbers of colonies in the soft agar. Taken together, this confirms functional inactivation of the transforming potential of the HPV16 antigens, and therefore the antigens can be considered as safe.

## Example 3: Immunogenicity of MfPV3 Ii-E1E2E6E7 in rhesus macaque monkeys

Materials and methods

*Cell lines, transfection and viral infection*

AGE1.CR.pIX and AGE1.CR.pIX-T-REx cells grown in suspension were maintained in chemically-defined CD-U6 medium (ProBioGen AG) supplemented with 2 mM L-glutamine (Sigma) and recombinant insulin-like growth factor (LONG-R$^3$ IGF, 50 ng/mL final concentration, Sigma) and were incubated in an orbital shaker (Multitron Pro,Infors HT) at 8% CO$_2$ with a shaking speed of 180 rpm. For adherent growth AGE1.CR.pIX and AGE1.CR.pIX-T-REx cells were cultivated in Dulbecco's Modified Eagle Medium DMEM/F12 supplemented with 5% Fetal Calf Serum (FCS). Adherent cell lines were maintained at 37°C and 8% CO$_2$ in a humidified incubator.

*Generation of recombinant MVA vectors*

Recombinant MVA vectors were generated as previously described (Jordan, I., Horn, D., Thiele, K., Haag, L., Fiddeke, K., Sandig, V., Virol Sin. 2020 Apr; 35(2): 212-226. doi: 10.1007/s12250-019-00176-3.). Briefly, the Genes of Interest (GOI) -(Figure 1) were cloned into the shuttle vector SP-CR19III-tetO, suitable for integration of the GOI into MVA's deletion site III under control of the MVA E/L-promoter. Recombinant MVA encoding the different GOIs were generated by homologous recombination in adherent AGE1.CR.pIX-T-REx cells that prevent the undesirable expression of the GOI during generation and propagation of the recombinant MVA by taking benefit of the Tet system (s. above). Therefore, the culture monolayers seeded in a six-well plate were infected with MVA or MVA-CR19 with a MOI of 0.05 and transfected with 2 $\mu$g of the individual shuttle vector using the Effectene Transfection Reagent (Qiagen, Germany) according to the manufacturer's instructions. The infected/transfected culture was harvested 2 -3 days post infection/transfection, sonicated, and used for infection of a cell monolayer in a six-well plate format. Resulting plaques were validated by PCR and an iterative plaque purification procedure was initiated until MVAs without the correct GOI expression cassette were not present any more (usually within 5-8 rounds of plaque purification).

For the propagation of plaque purified MVAs the cell harvest material was sonicated using a Vial Tweeter (set to 20 s of 100% cycle and 90% amplitude, Hielscher, Germany), and AGE1.CR.pIX-T-REx (grown in suspension at $2\times 10^6$ cells per ml in a 1:1 mixtures of CD-U4 and CD-VP4 (Merck-Millipore) were inoculated with the individual recombinant MVA vectors at MOI 0.05. Finally, MVAs were harvested 48 h - 72 h post infection and the $TCID_{50}$ titer was determined.

*Animals*

Ethical approval for use of cynomolgus macaques was granted by the primate research center at the Bogor Agricultural University IACUC (Animal Care and Use Committee). Female cynomolgus macaques between 6 and 10 years old and weighing between 3 and 5 kg were selected for use in this study based on negativity for tuberculosis, Simian T-cell leukemia virus and Simian retrovirus and cervical positivity for MfPV3. The selected animals were acclimatized for a month before vaccination. Animals were housed in groups and fed with monkey chow (Charoen Pokpand, Thailand) twice a day.

All procedures were performed by highly trained veterinarians, animal technicians and caretakers. Before all procedures such as vaccination and blood withdrawal, cervical swabs, animals were anesthetized with Ketamine (10-15mg/ kg) and Xylazine (0.5—1mg/kg). Before and after the procedures, macaques were monitored for pain, distress, body temperature and weight. Blood collection was limited to the maximum volume allowed over the duration of the experiment. The experiment is still ongoing, but all animals were returned into the colony at the end of the study for use in other experiments or breeding programs.

*DNA Purification from Cervical Swabs and MfPV3 detection by qPCR*

Briefly, cervical samples were obtained using a small cervix cytobrush and placed in a preservative solution called TEN buffer (Tris HCl 10 mM pH 7.5; EDTA 5 mM, NaCl 50 mM). DNA was extracted from the cervical samples with the DNA extraction kit (QIAmp DNA blood mini kit, Qiagen, Hilden, Germany) and eluted in 100 mL following the manufacturer's procedure.

Selection of MfPV3 positive cynomolgus macaques, and detection of MfPV3 infection level during the therapeutic vaccination trial was performed at Bogor University (Indonesia) by quantitative PCR (qPCR) with the following set of primers and probe: 5'FAM-aaatggtgcagtgggcatacgacc-3'BHQ1, gggaaccacctgaatggata and ggcgcaatccttcacatatt.

*Vaccinations*

Animals were vaccinated on day 0 with either PBS or Ad19a/64 vectored MfPV3 Ii-E1E2E6E7 and with MVA vectored MfPV3 Ii-E1E2E6E7 on day 42. Vaccines were given both i.m., i.rec. and i.vag, $2\times 10^8$ IFU at each route.

*ELISpot*

Peripheral blood mononuclear cells (PBMCs) from each vaccinated cynomolgus macaques were purified from blood by Ficoll-Paque (Sigma). Vaccine antigen specific T-cells from the fresh purified PBMCs were measured by IFN-$\gamma$ production using the Monkey IFN-$\gamma$ ELISPOTplus kit (MABTECH, #3421M-4HST-2) after 18-20 hours incubation at 37 C and 5% CO2 in presence of 2 mg/mL of the relevant peptide pools. Spot-forming units (SFU) were read on an IRIS instrument and counted using the ELISpot Big emphasis algorithm. Background responses from unstimulated samples were subtracted prior to graphical representation.

*Flow cytometry of NHP PBMCs*

Peripheral blood mononuclear cells (PBMCs) from each vaccinated cynomolgus macaques were purified from blood by Ficoll-Paque (Sigma). Cells were incubated for 5 hours in 20 ug/uL brefeldin A with or without 2 $\mu$g/mL of relevant peptides. The cells were stained against surface markers: APC-CD3 (clone SP34-2, BD biosciences), Per-CP/Cy5.5-CD4 (Clone L200, BD biosciences), PE-CD8 (Clone RPA-T8, BD biosciences) and fixable viability dye eFlour780 (Thermofisher). After surface staining, cells were fixed and permeabilized in BDcytofix/Cytoperm, and stained intracellularly using FITC-IFN-$\gamma$ (Clone MD-1, UCy-Tech) and PE/Cy7-TNF-$\alpha$ (Clone mAb11, BD biosciences) antibodies.

The peptides used were 16-mers overlapping by 11 amino acids covering the entire Ii-E1E2E6E7 antigen. The peptides were pooled in 5 separate pools containing Ii, E1, E2, E6 and E7 peptides respectively. Peptides were obtained from KareBay, Town, China.

Flow cytometry was performed on FACS CANTO flow cytometer and data analysis was performed using FlowJo V10 software. Epitope-specific CD8$^+$ T-cell responses were measured as viable CD3$^+$, CD8$^+$ or CD4$^+$, IFN-$\gamma^+$ cells. Background (from unstimulated samples) were subtracted before plotting and analysis. All responses below 0.01% were regarded as below detection limit, and manually adjusted to 0.01 for optimal visual representation.

Results

*Responses after prime vaccination measured by ELISpot*

All vaccinated animals respond strongly to the E1 peptide pool (figure 10), compared to the PBS (sham/negative control) vaccinated animals. There are E2 responses in all vaccinated animals, albeit at a lower frequency than E1, and E6/E7 responses are seen in two of the vaccinated animals.

This provides evidence that the Ii-E1E2E6E7 Ad19a/64 vaccine is immunogenic in monkeys.

*Responses after boost vaccination, measured by intracellular staining and flow cytometry*

Very low T-cell responses are detected prior to boost (figure 11, top panels), but 9 days post MVA boost, the T-cell responses have been re-amplified. All vaccinated monkeys show strong CD8 and CD4 responses against E1 in contrast to the PBS controls, and CD8 responses against E2 and E7 are detected in a number of vaccinated animals as well (Figure 10, bottom panels).

Besides confirming that the viral vectored MfPV3 Ii-E1E2E6E7 vaccine is immunogenic in MfPV3 infected monkeys, this also provides a strong indication that the vaccine can cure the pre-existing MfPV3 infections in the NHPs.

*Clearance of MfPV3 infection at day 56 in all vaccinated animals*

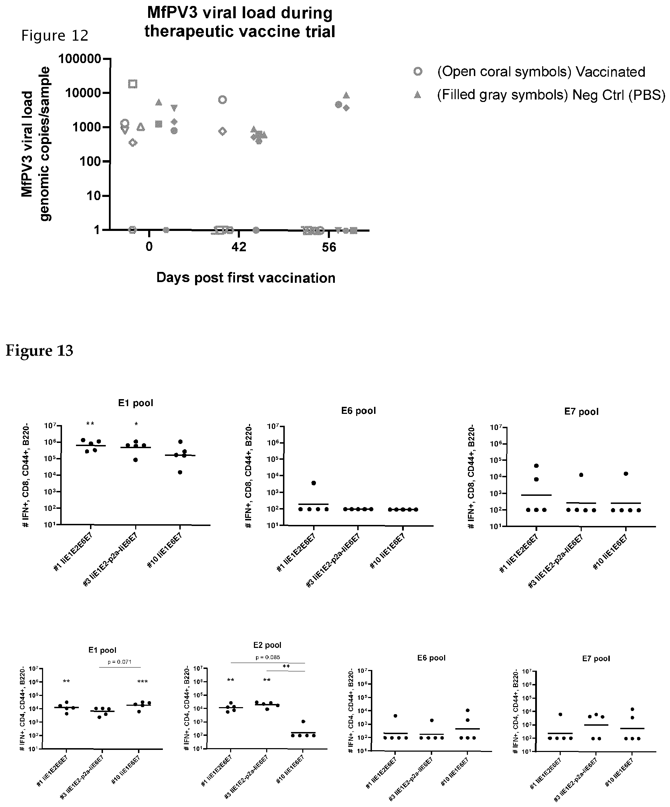

As seen in figure 12, only two of the vaccinated animals have detectable MfPV3 vaccination at day 42 (6 weeks after prime) and all vaccinated animals have cleared the infection at day 56 (2 weeks after boost) compared to only 3 out of 6 of the PBS injected negative control animals. Note that one animal in the vaccine-group and one animal of the negative ctrl-group did not have detectable MfPV3 at day 0 (each symbol represents one animal). However, the negative ctrl animal with no detectable infection at day 0, had detectable viremia at day 42, and is therefore regarded as infected, meaning that the clearance rates are 5/5 and 3/6 for the vaccine- and PBS-groups respectively, resulting in a p-value = 0.18 (Fishers's Exact test).

**Example 4: properties of the different construct designs with HPV16 E1, E2, E6 and E7 antigens, and tumour protective efficacy of HPV16 Ii-E1E2E6E7**

Materials and methods:

*Animals, immunizations, cisplatin injections and tumour challenges*

C57BL/6, Hsd-Ola and CD1 female mice were obtained from Envigo (Horst, The Netherlands). OF1 mice All animals were housed at the Panum Institute, University of Copenhagen. All experiments were initiated after allowing the mice to acclimatize for at least 1 wk. Experiments were approved by the National Animal Experiments Inspectorate (Dyreforsøgstilsynet, license no. 2016-15-0201-01131) and performed according to national guidelines.

Immunizations with adenoviral vectors were performed intramuscular (i.m.) with $1\times10^7$ TCID50 MVA or with $2\times10^7$ IFU Ad19a/64 or Ad5 diluted in 50 $\mu$L PBS. Mice were anesthetized with isofluorane before injections.

Cisplatin was dissolved to 1 mg/mL in 0.9% NaCl2 solution, and injected as 3 mg/kg i.p..

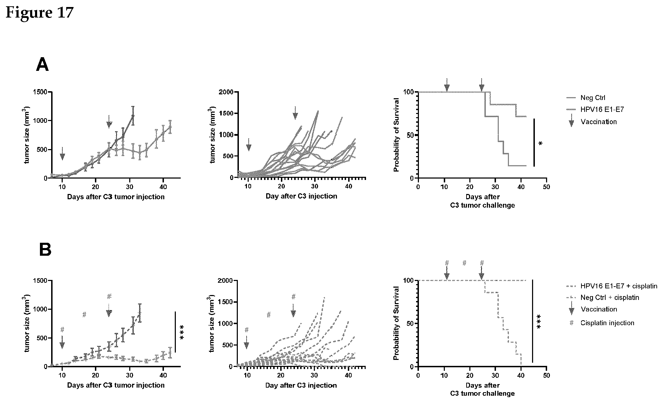

The tumour cell line C3 was developed by transfection of mouse embryonic cells with the HPV16 genome and an activated ras oncogene (Feltkamp et al, Eur J Immunol 1993). Cell line authentication was carried out by flow cytometry detection of HPV16 E7 presence. To assess tumour growth in vivo, $1 \times 10^6$ C3 cells in 100 μL PBS+0.2%BSA were injected s.c. into the right thigh of C57BL/6 mice. Tumour size was measured three times a week in length and width and the tumour volume was calculated as: length * width$^2$ * 0.5236 (Janik et al, Cancer Res 1975). Mice were euthanized when tumours exceeded 2000 mm$^3$ (Figure 17) or 1000 mm$^3$ (Figure 18), necrotic wounds emerged or mobility of the mice was markedly reduced.

*Flow cytometry of splenocytes*

Single cell suspensions of splenocytes were obtained by organ harvest in HANKs followed by straining through 70 μm cell strainers. Cells were incubated for 5 hours in 3 μM monensin with or without 1 μg/mL of relevant peptides. The cells were stained against surface markers: APC-Cy7 or BV421 CD8 (53-6.7, 1:200, BioLegend, San Diego, USA), PE-Cy7 CD4 (RM4-5, 1:800, BD), FITC CD44 (IM7, 1:100, BioLegend, San Diego, USA) and PerCP-Cy5.5 B220 (RA3-6B2, 1:200, BioLegend, San Diego, USA). After surface staining, cells were fixed in 1% PFA, permeabilized in 0.5% saponine, and stained intracellularly using APC IFN-γ (XMG1.2, 1:100, BioLegend, San Diego, USA) and PE TNF-α (MP6-XT22, 1:100, BioLegend, San Diego, USA) antibodies.

The peptides used were 16-mers overlapping by 11 amino acids covering the entire Ii-E1E2E6E7 antigen. The peptides were pooled in 5 separate pools containing Ii, E1, E2, E6 and E7 peptides respectively. Peptides were obtained from KareBay, Town, China.

Flow cytometry was performed on the Fortessa 3 (BD Biosciences, Franklin Lakes, USA) flow cytometer and data analysis was performed using FlowJo V10 software. Epitope-specific CD8$^+$ T-cell responses were measured as B220-, CD8$^+$ or CD4$^+$, CD44$^+$, IFN-γ$^+$ cells and are presented in total number of cells per organ. The quality of the IFN-γ$^+$ responses were evaluated by MFI of IFN-γ and fraction of double positive cells (expressing both IFN-γ and TNF) in the IFN-γ$^+$ CD8$^+$ or CD4$^+$ populations. The gating strategy is shown in Supplementary figure 1.

Results

*Immune responses in mice after Ad19a/64 HPV16 Ii-E1E2E6E7 vaccination*

Outbred CD1 mice were vaccinated on day 0 with Ad19a/64 HPV16 Ii-E1E2E6E7, Ii-E1E2-p2a-Ii-E6E7 or Ii-E1E6E7, and on day 21 with rAd5 formulation of same constructs. Splenic T-cell responses were analysed on day 31 by intracellular staining and flow cytometry. CD8 T-cell responses were detected against E1, E6 and E7 for Ii-E1E2E6E7 and CD4 responses against E1, E2, E6 and E7 (figure 13). This supports the findings in example 1, that all constructs are immunogenic, and that the preferred order of the antigens is E1 followed by E2, E6 and E7.

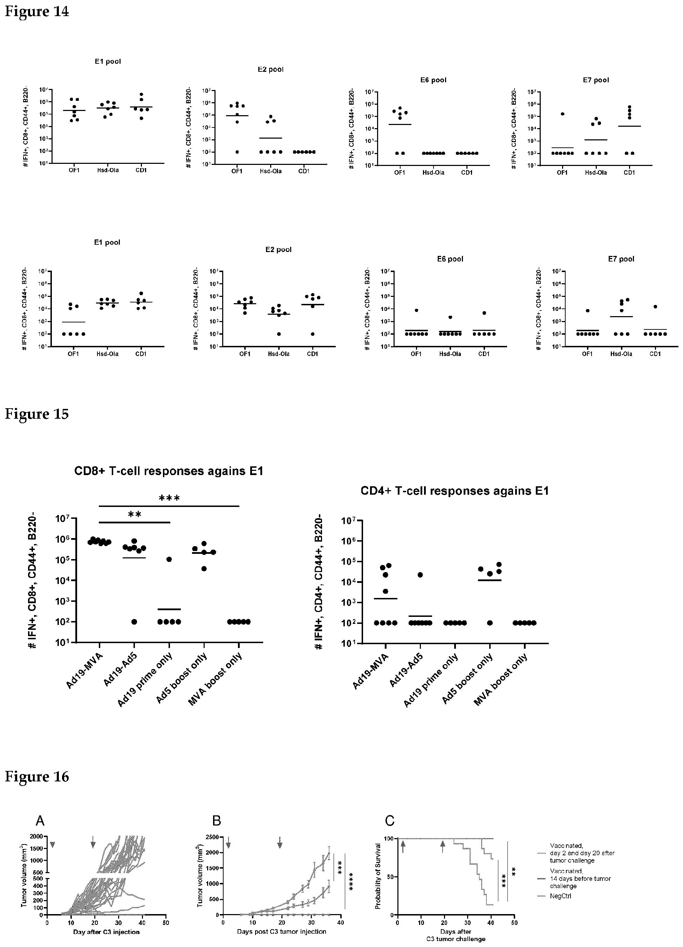

To ensure that also the Ad19 prime - MVA boost vaccination regimen was immunogenic, outbred OF1, CD1 or Hsd-Ola mice were vaccinated on day 0 with Ad19a/64 HPV16 Ii-E1E2E6E7 and on day 28 with MVA HPV16 Ii-E1E2E6E7. It was seen, that the Ad19-MVA regimen elicited immune responses against all four vaccine antigens at 10 days post boost (figure 14). Furthermore, inbred C57BL/6 mice were given booster vaccinations with either Ad5 or MVA HPV16 Ii-E1E2E6E7 on day 28 for direct comparison. The immune responses against HPV16 E1 10 days after boost clearly shows that the MVA boosting Ad5 and MVA boosting gives comparable T-cell responses (figure 15).

*Tumour efficacy of HPV16 Ii-E1E2E6E7 vaccination*

Inbred C57BL/6 mice were challenged with 1E+06 HPV16+ C3 tumour cells s.c. in the flank. Mice were wither vaccinated with Ad19a/64 Ii-E1E2E6E7 14 days before tumour challenge (blue), vaccinated with Ad19a/64 HPV16 Ii-E1E2E6E7 two days after tumour challenge, and with Ad5 HPV16 Ii-E1E2E6E7 20 days after C3 injection (coral), or with Ad19a/64 and Ad5 vectors encoding Ii followed by an irrelevant antigen as negative controls (gray). The tumour growth was followed over time (figure 16 A (individual mice) and B (mean+SEM)), and mice were sacrificed when tumour sizes exceeded 2000 mm3 (figure 16 C).

Vaccination shortly after tumour challenge significantly reduces tumour growth and increases survival. Furthermore, prophylactic vaccination completely protects against tumour growth, indicating that patients treated with this vaccine, would be protected from relapse.

To investigate the effect of therapeutic vaccination on more established tumors, a similar experiment as above was performed but with the vaccinations given on day 10 and day 14 instead of day 2 and 20. As cisplatin has is commonly used in the clinic to treat HPV+ cancers, some mice received three weekly cisplatin (3mg/kg bodyweight) injection starting on day 10.

As seen in figure 17 A, the vaccinations alone are able to abrogate tumor growth after the booster immunization, and furthermore figure 17 B shows how vaccination and cisplatin together controls tumor growth completely. The vaccine significantly improves survival alone and especially in combination with cisplatin.

**Claims**

1. A nucleic acid comprising or consisting of nucleic acid sequences encoding papilloma virus proteins E1, E6, and E7; wherein said nucleic acid molecule encodes a single polyprotein.

2. The nucleic acid of claim 1, furthermore comprising or further consisting of a nucleic acid sequence encoding papilloma virus protein E2.

3. The nucleic acid of claim 1 or 2, wherein the order of the nucleic acids encoding said proteins is, from 5' to 3',

   (a) to the extent E2 is present

      (i) E1, E2, E6, E7; or
      (ii) E1, E6, E7, E2;

   or
   (b) E1, E6, E7.

4. A nucleic acid, preferably of any one of claims 1 to 3, comprising or consisting of nucleic acid sequences encoding papilloma virus proteins E6 and/or E7, wherein said nucleic acid sequences are modified as compared to the wild-type counterparts by point mutations and/or deletions, wherein said point mutations and/or deletions

   (i) abolish colony forming in a soft agar assay; and
   (ii) said modified proteins E6 and E7 maintain at least 70 % of predicted high-affinity epitopes (IC50 < 50 nM) and at least 50 %, at least 55% or at least 58% of predicted low-affinity epitopes (50 nM < IC50 < 5 $\mu$M) of the predicted epitopes of the respective wild-type protein.

5. The nucleic acid of any one of the preceding claims, wherein said papilloma virus is a primate papilloma virus, preferably a human papilloma virus (HPV) such as HPV16, HPV18, HPV45, HPV31, HPV33, HPV35, HPV52, HPV58, HPV6 and HPV11, more preferably HPV16 or HPV18, or macaque papilloma virus, preferably MfPV3.

6. The nucleic acid of claim 4 or 5, wherein only the following modifications as compared to the wild-type are present in the encoded proteins:

   (iii) in E6: a modification which reduces or abolishes interactions mediated by the PDZ domain comprised in wild-type E6; and

      a. in HPV16 E6: $L117X_1$;
      b. in HPV18 E6: $L112X_1$;
      c. in MfPV3 E6: $L110X_1$;

   and
   (iv) in E7:

      a. in HPV16 E7: $C24X_2$, $L67X_3$, and $C91X_4$;
      b. in HPV18 E7: $C27X_2$, $L74X_3$, and $C98X_4$;
      c. in MfPV3 E7: $C24X_2$, $L71X_3$, and $C95X_4$;

   wherein $X_1$ to $X_4$ are independently a proteinogenic amino acid different from the respective amino acid they substitute.

7. The nucleic acid of claim 6, wherein said nucleic acid furthermore comprises one or both mutations encoding $D21X_5$ and $C58X_6$ in HPV 16 E7, $D24X_5$ and $C65X_6$ in HPV 18 E7, and $D21X_5$ and $C62X_6$ in MfPV3 E7, wherein $X_5$ and $X_6$ are independently a proteinogenic amino acid different from the respective amino acid they substitute.

8. The nucleic acid of claim 6 or 7, wherein

(i) $X_1$ is selected from Q, Y, and N, and preferably is Q;
(ii) $X_2$ is selected from G, A, V, L, and I, and preferably is G;
(iii) $X_3$ is selected from R, K, and H, and preferably is R;
(iv) $X_4$ is selected from A, G, V, L, and I, and preferably is A;
(v) $X_5$ is selected from G, A, V, L, and I, and preferably is G;
(vi) $X_6$ is selected from G, A, V, L, and I, and preferably is G; and/or
(vii) said modification which reduces or abolishes interactions mediated by the PDZ domain is a deletion of 1 to 10 C-terminal amino acids, preferably counted from the C-terminus, more preferably ΔETQL in HPV16 E6, ΔETQV in HPV18 E6, and ΔETEV in MfPV3 E6; or said modification are one, two, three or four point mutation with the 10, preferably the four C-terminal amino acids.

9. The nucleic acid of any one of claims 1 to 8, wherein the total number of Cys residues in said polyprotein is an even number.

10. The nucleic acid of claim 9, wherein if said number is otherwise not even,

(ii) a sequence encoding

a. HPV16 E2 with a C300$X_7$ mutation;
b. HPV18 E2 with a C301$X_7$ mutation; or
c. MfPV3 E2 with a C297$X_7$ mutation
is comprised in said nucleic acid as sequence encoding E2, wherein $X_7$ is a proteinogenic amino acid other than C, preferably selected from A, S, G, M, T, Y, Q, N, V, L, I, F, W, H, K, R, Q, N, and P, more preferably A;

(iii) or a codon encoding Cys is inserted in said nucleic acid.

11. The nucleic acid of any one of the preceding claims, wherein said nucleic acid furthermore comprises at least one nucleic acid sequence encoding a genetic adjuvant, preferably T cell adjuvant MHC-II-associated invariant chain.

12. The nucleic acid of claim 11, wherein said invariant chain

(i) is present once and precedes the sequence encoding the first E protein; or
(ii) is present twice, one copy preceding the sequence encoding the first E protein, and a second copy preceding the sequence encoding E6 or, to the extent present, the sequence encoding E2.

13. The nucleic acid of any one of the preceding claims, wherein said nucleic is

(i) DNA;
(ii) a plasmid; or
(iii) RNA, preferably mRNA.

14. The nucleic acid of claim 13(i), wherein said nucleic acid comprises an untranslated 5' terminus comprising a Tet operator.

15. The nucleic acid of any one of the preceding claims, wherein said nucleic acid comprises at least one nucleic acid sequence encoding a self-cleaving peptide, wherein said nucleic acid sequence encoding a self-cleaving peptide

(i) precedes at least one nucleic acid sequence encoding a genetic adjuvant; or
(ii) is located between and adjacent to two nucleic acid sequences encoding a papilloma virus protein.

16. The nucleic acid of any one of the preceding claims, wherein neighbouring nucleic acid sequences are connected in one, more or all instances by sequences encoding a flexible linker which is 10 or less amino acids in length such as 2, 3, 4, or 5 amino acids, wherein GS and GCS are preferred linkers.

17. A viral vector comprising the nucleic acid of any one of the preceding claims.

18. The viral vector of claim 17, wherein said vector is a DNA viral vector, preferably

(i) an adenoviral vector, preferably being deficient with regard to adenoviral E1 and E3 encoding sequences, said vector more preferably being Ad19a/64, Ad5, Ad5 with fiber replacements such as Ad5F35, Ad26, Chimp63, or chAdOx1; or
(ii) a Poxvirus vector, preferably MVA, MVA-CR19, SCV, Vaccinia, or Fowlpox.

19. A polyprotein encoded by the nucleic acid or the vector of any of the preceding claims or cleavage products derived therefrom.

20. A pharmaceutical composition, preferably a vaccine, comprising or consisting of

(i) a nucleic acid of any one of claims 1 to 16;
(ii) a vector of claim 17 or 18;
(iii) a polyprotein or cleavage products thereof of claim 19; or
(iv) a cell transduced with the nucleic acid of (i) or the vector of (ii), wherein preferably said cell is an ex vivo or in vitro cell and/or a dendritic cell or a monocyte.

21. The pharmaceutical composition of claim 20, wherein said vector, said nucleic acid, said polyprotein or said cell is the only pharmaceutically active agent.

22. The pharmaceutical composition of claim 21, wherein said pharmaceutical composition comprises two or more pharmaceutically active agents, wherein a second pharmaceutically active agent is selected from

(i) a second nucleic acid of any one of claims 1 to 16;
(ii) a second vector of claim 17 or 18;
(iii) a second polyprotein or cleavage products of claim 19;
(iv) a second cell as defined in claim 20 (iv);
(v) a protein-based vaccine, preferably against papilloma virus; and
(vi) a chemotherapeutic, preferably cisplatin.

23. The pharmaceutical composition of claim 22 (ii), wherein said pharmaceutical composition comprises two pharmaceutically active agents which are

(i) an adenoviral vector as defined in claim 18 (i); and
(ii) a Poxvirus vector as defined in claim 18 (ii),
wherein said two pharmaceutically active agents are preferably confectioned as two pharmaceutical compositions which may be administered in any order or concomitantly, preferably the pharmaceutical composition comprising said Poxvirus vector after said pharmaceutical composition comprising said adenoviral vector, preferably after at least 4 weeks have elapsed.

24. The nucleic acid, the vector, the polyprotein, or the cell of any one of the preceding claims for use in medicine.

25. The vaccine of claim 20, wherein said vaccine is a therapeutic vaccine.

26. The nucleic acid, the vector, the polyprotein, the cell, or the vaccine of any of the preceding claims for use in a method of treating or preventing papilloma virus infection, low-grade squamous intraepithelial lesions (LSIL), high-grade squamous intraepithelial lesions (HSIL), nasal polyposis or papilloma virus-associated malignant conditions such as cervical cancer, head and neck squamous cell carcinoma (HNSCC), and oropharyngeal squamous cell carcinoma (OPSCC).

27. An in vitro or ex vivo method of stimulating and/or expanding T cells, said method comprising bringing into contact in vitro or ex vivo T cells with a nucleic acid, a vector, or a polyprotein of any one of the preceding claims.

## Figure 1

## Figure 2

Figure 3

EP 4 137 153 A1

Figure 4

**A**

**C**

**B**

Figure 5

Figure 6

A

li-E1E2E6E7　E1E2E6E7　empty plasmid　li-E1E2E6E7　E1E2E6E7　empty plasmid

MG132　+　+　+　−　−　−

kDa

180
130
100
70
55

anti-ubiquitin

B

180
130

anti-myc

C

70
55

anti-tubulin

D

li-E1-SIINFEKL　E1-SIINFEKL　empty plasmid　li-E1-SIINFEKL　E1-SIINFEKL　empty plasmid

MG132　−　−　−　+　+　+

kDa

180
130
100
70
55

anti-myc

E

70
55

anti-tubulin

Figure 7

Figure 8

**Figure 9**

E6 alignments:

E7 alignments:

Figure 9, continued

E2 alignment:

Figure 10

**IFN-γ producing cells in NHP PBMCs
2 weeks after Ad19a prime immunization**

Figure 11

Figure 12

**MfPV3 viral load during therapeutic vaccine trial**

○ (Open coral symbols) Vaccinated
▲ (Filled gray symbols) Neg Ctrl (PBS)

Figure 13

Figure 14

Figure 15

Figure 16

Figure 17

Europäisches
Patentamt

European
Patent Office

Office européen
des brevets

# EUROPEAN SEARCH REPORT

Application Number

EP 21 19 1940

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| T | NECKERMANN PATRICK ET AL: "Design and Immunological Validation of Macaca fascicularis Papillomavirus Type 3 Based Vaccine Candidates in Outbred Mice: Basis for Future Testing of a Therapeutic Papillomavirus Vaccine in NHPs", FRONTIERS IN IMMUNOLOGY, vol. 12, 28 October 2021 (2021-10-28), XP055914517, DOI: 10.3389/fimmu.2021.761214 * the whole document * | | INV. A61K39/12 A61P31/20 A61P35/00 C07K14/005 |
| Y | BRANDSMA JANET L ET AL: "Vesicular stomatitis virus-based therapeutic vaccination targeted to the E1,E2, E6, and E7 proteins of cottontail rabbit papillomavirus", JOURNAL OF VIROLOGY, THE AMERICAN SOCIETY FOR MICROBIOLOGY, US, vol. 81, no. 11, 1 June 2007 (2007-06-01), pages 5749-5758, XP009089830, ISSN: 0022-538X *** pages 5749, 5750, Figure 1 *** | 1-27 | |
| Y | BRANDSMA ET AL: "Therapeutic vaccination of rabbits with a ubiquitin-fused papillomavirus E1, E2, E6 and E7 DNA vaccine", VACCINE, ELSEVIER, AMSTERDAM, NL, vol. 25, no. 33, 24 July 2007 (2007-07-24) , pages 6158-6163, XP022168615, ISSN: 0264-410X, DOI: 10.1016/J.VACCINE.2007.06.012 *** abstract, chapter 2.2, Figure 2 *** | 1-27 | |

TECHNICAL FIELDS SEARCHED (IPC)

A61K
A61P
C07K
C12N

-/--

The present search report has been drawn up for all claims

| Place of search | Date of completion of the search | Examiner |
|---|---|---|
| The Hague | 12 May 2022 | Heder, Andreas |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

................................................................

& : member of the same patent family, corresponding document

Europäisches
Patentamt

European
Patent Office

Office européen
des brevets

# EUROPEAN SEARCH REPORT

**Application Number**

EP 21 19 1940

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | LEACHMAN SANCY A ET AL: "Ubiquitin-fused and/or multiple early genes from cottontail rabbit papillomavirus as DNA vaccines", JOURNAL OF VIROLOGY, THE AMERICAN SOCIETY FOR MICROBIOLOGY, US, vol. 76, no. 15, 1 August 2002 (2002-08-01), pages 7616-7624, XP002415253, ISSN: 0022-538X, DOI: 10.1128/JVI.76.15.7616-7624.2002 ----- | 1-27 | |
| A,D | RAGONNAUD EMELINE ET AL: "Replication deficient human adenovirus vector serotype 19a/64: Immunogenicity in mice and female cynomolgus macaques", VACCINE, ELSEVIER, AMSTERDAM, NL, vol. 36, no. 41, 3 September 2018 (2018-09-03), pages 6212-6222, XP085480649, ISSN: 0264-410X, DOI: 10.1016/J.VACCINE.2018.07.075 *** chapter 2.2 *** ----- | 1-27 | |
| A | US 2005/118139 A1 (HUANG LINGYI [US] ET AL) 2 June 2005 (2005-06-02) ----- | 1-27 | **TECHNICAL FIELDS SEARCHED (IPC)** |

The present search report has been drawn up for all claims

| Place of search | Date of completion of the search | Examiner |
|---|---|---|
| The Hague | 12 May 2022 | Heder, Andreas |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons
...............................................................................
& : member of the same patent family, corresponding
document

EPO FORM 1503 03.82 (P04C01)

Europäisches
Patentamt

European
Patent Office

Office européen
des brevets

**Application Number**

**EP 21 19 1940**

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

## LACK OF UNITY OF INVENTION

The Search Division considers that the present European patent application does not comply with the requirements of unity of invention and relates to several inventions or groups of inventions, namely:

**see sheet B**

☐ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☒ None of the further search fees have been paid within the fixed time limit. The present European search report has been drawn up for those parts of the European patent application which relate to the invention first mentioned in the claims, namely claims:

**1-3(completely); 4-27(partially)**

☐ The present supplementary European search report has been drawn up for those parts of the European patent application which relate to the invention first mentioned in the claims (Rule 164 (1) EPC).

**LACK OF UNITY OF INVENTION**
**SHEET B**

The Search Division considers that the present European patent application does not comply with the requirements of unity of invention and relates to several inventions or groups of inventions, namely:

1. claims: 1-3(completely); 4-27(partially)

   Nucleic acid encoding single polyprotein comprising or consisting of papillomavirus E1, E6, E7, and, optionally, E2, vectors, pharmaceutical compositions comprising the same, medical use thereof
   ---

2. claims: 4-27(partially)

   Nucleic acid encoding modified papillomavirus E6 and / or E7, vectors, pharmaceutical compositions comprising the same, medical use thereof
   ---

## ANNEX TO THE EUROPEAN SEARCH REPORT
## ON EUROPEAN PATENT APPLICATION NO.

EP 21 19 1940

This annex lists the patent family members relating to the patent documents cited in the above-mentioned European search report.
The members are as contained in the European Patent Office EDP file on
The European Patent Office is in no way liable for these particulars which are merely given for the purpose of information.

12-05-2022

| Patent document cited in search report | | Publication date | Patent family member(s) | | Publication date |
|---|---|---|---|---|---|
| US 2005118139 | A1 | 02-06-2005 | CA | 2457890 A1 | 06-03-2003 |
| | | | EP | 1427443 A1 | 16-06-2004 |
| | | | US | 2005118139 A1 | 02-06-2005 |
| | | | WO | 03018055 A1 | 06-03-2003 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2007062656 A
- WO 2010057501 A
- WO 2018172259 A
- WO 2020079234 A
- WO 2015082922 A

### Non-patent literature cited in the description

- **SUNG, H. et al.** Global Cancer Statistics 2020: GLOBOCAN Estimates of Incidence and Mortality Worldwide for 36 Cancers in 185 Countries. *CA: A Cancer Journal for Clinicians,* 2001, vol. 71 (3), 209-249
- **HAGHSHENAS, M. et al.** Prevalence and type distribution of high-risk human papillomavirus in patients with cervical cancer: a population-based study. *Infect. Agent. Cancer,* 20 August 2013
- **BROTHERTON, J. M. L. ; ZUBER, P. L. F. ; BLOEM, P. J. N.** Primary Prevention of HPV through Vaccination: Update on the Current Global Status. *Curr. Obstet. Gynecol. Rep.,* 2016, vol. 5, 210-224
- **LEHTINEN, M. et al.** Overall efficacy of HPV-16/18 AS04-adjuvanted vaccine against grade 3 or greater cervical intraepithelial neoplasia: 4-year end-of-study analysis of the randomised, double-blind PATRICIA trial. *Lancet. Oncol.,* 2012, vol. 13, 89-99
- **HILDESHEIM, A. et al.** Effect of human papillomavirus 16/18 L1 viruslike particle vaccine among young women with preexisting infection: a randomized trial. *JAMA,* 2007, vol. 298, 743-753
- **SCHILLER, J. T. ; CASTELLSAGUÉ, X. ; GARLAND, S. M.** A review of clinical trials of human papillomavirus prophylactic vaccines. *Vaccine,* 2012, vol. 30 (5), F123-38
- **HUNG, C.-F. ; MA, B. ; MONIE, A. ; TSEN, S.-W. ; WU, T.-C.** Therapeutic human papillomavirus vaccines: current clinical trials and future directions. *Expert Opin. Biol. Ther.,* 2008, vol. 8, 421-439
- **HO, G. Y. ; BIERMAN, R. ; BEARDSLEY, L. ; CHANG, C. J. ; BURK, R. D.** Natural history of cervicovaginal papillomavirus infection in young women. *N. Engl. J. Med.,* 1998, vol. 338, 423-428
- **XUE, Y. et al.** HPV16 E2 is an immediate early marker of viral infection, preceding E7 expression in precursor structures of cervical carcinoma. *Cancer Res.,* 2010, vol. 70, 5316-5325
- **CHANG, L. ; HE, X. ; YU, G ; WU, Y.** Effectiveness of HPV 16 viral load and the E2/E6 ratio for the prediction of cervical cancer risk among Chinese women. *J. Med. Virol.,* 2013, vol. 85, 646-654
- **HIBMA, M. H.** The Immune Response to Papillomavirus During Infection Persistence and Regression. *The Open Virology Journal,* 2012, vol. 6
- **MONNIER-BENOIT, S. et al.** Immunohistochemical analysis of CD4+ and CD8+ T-cell subsets in high risk human papillomavirus-associated pre-malignant and malignant lesions of the uterine cervix. *Gynecol. Oncol.,* 2006, vol. 102, 22-31
- **DE JONG, A. et al.** Human Papillomavirus Type 16-Positive Cervical Cancer Is Associated with Impaired CD4+ T-Cell Immunity against Early Antigens E2 and E6. *Cancer Res.,* 2004, vol. 64, 5449 LP-5455
- **WOO, Y. L. et al.** A prospective study on the natural course of low-grade squamous intraepithelial lesions and the presence of HPV16 E2-, E6- and E7-specific T-cell responses. *Int. J. cancer,* 2010, vol. 126, 133-141
- **RAGONNAUD, E. ; PEDERSEN, A. G. ; HOLST, P. J.** Breadth of T Cell Responses After Immunization with Adenovirus Vectors Encoding Ancestral Antigens or Polyvalent Papillomavirus Antigens. *Scand. J. Immunol.,* 2017, vol. 85, 182-190
- **DILLON, S. et al.** Resolution of cervical dysplasia is associated with T-cell proliferative responses to human papillomavirus type 16 E2. *J. Gen. Virol.,* 2007, vol. 88, 803-813
- **KOSKIMAA, H. M. et al.** Human papillomavirus 16-specific cell-mediated immunity in children born to mothers with incident cervical intraepithelial neoplasia (CIN) and to those constantly HPV negative. *J. Transl. Med.,* 2015, vol. 13, 1-11
- **CHEN, Z. et al.** Non-human Primate Papillomaviruses Share Similar Evolutionary Histories and Niche Adaptation as the Human Counterparts. *Front. Microbiol.,* 2019, vol. 10, 2093
- **CHEN, Z. et al.** Genomic diversity and interspecies host infection of alpha12 Macaca fascicularis papillomaviruses (MfPVs). *Virology,* 2009, vol. 393, 304-310

- **WOOD, C. E. ; CHEN, Z. ; CLINE, J. M. ; MILLER, B. E. ; BURK, R. D.** Characterization and Experimental Transmission of an Oncogenic Papillomavirus in Female Macaques. *J. Virol.,* 2007, vol. 81, 6339-6345
- **NANCE ; MEIER.** *ACS Cent Sci.,* 26 May 2021, vol. 7 (5), 748-756
- **BERGVALL et al.** *Virology,* October 2013, vol. 445 (1,2), 35-56
- **VANDE POL ; KLINGELHUTZ.** *Virology,* October 2013, vol. 445 (1,2), 115-37
- **ROMAN ; MUNGER.** *Virology,* 2013, vol. 445, 138-68
- **MCBRIDE.** *Virology,* October 2013, vol. 445 (1,2), 57-79
- **NIELSEN et al.** *Protein Sci,* 2003, vol. 12, 1007
- **LUNDEGAARD et al.** *Nucl Acids Res,* 2008, vol. 36, W509
- **ANDREATTA et al.** *Bioinformatics,* 2016, vol. 32, 511
- **HU et al.** *Signal Transduction and Targeted Therapy,* 2020, vol. 5, 101
- **HILLEN W. ; BERENS.** Mechanisms underlying expression of Tn10 encoded tetracycline resistance. *Annu Rev Microbiol.,* 1994, vol. 48, 345-69
- **W HILLEN ; C GATZ ; L ALTSCHMIED ; K SCHOLLMEIER ; I MEIER.** Control of expression of the Tn10-encoded tetracycline resistance genes. Equilibrium and kinetic investigation of the regulatory reactions. *J Mol Biol.,* 25 September 1983, vol. 169 (3), 707-21
- **KATHLEEN POSTLE ; TOAI T. NGUYEN ; KEVIN P. BERTRAND.** Nucleotide sequence of the repressor gene of the TN10 tetracycline resistance determinant. *Nucleic Acids Research,* 25 June 1984, vol. 12 (12), 4849-4863
- **FENG YAO ; TOR SVENSJÖ ; THOMAS WINKLER ; MICHAEL LU ; CARL ERIKSSON ; ELOF ERIKSSON.** Tetracycline Repressor, tetR, rather than the tetR-Mammalian Cell Transcription Factor Fusion Derivatives, Regulates Inducible Gene Expression in Mammalian Cells. *Human Gene Therapy.,* September 1998, 1939-1950
- **SCHIEDNER G ; HERTEL S ; KOCHANEK S.** *Hum Gene Ther.,* 10 October 2000, vol. 11 (15), 2105-16
- **ZHOU X. ; ROBINSON C.M. ; RAJAIYA J. ; DEHGHAN S. ; SETO D. ; JONES M.S. ; DYER D.W. ; CHODOSH J.** Analysis of human adenovirus type 19 associated with epidemic keratoconjunctivitis and its reclassification as adenovirus type 64. *Invest. Ophthalmol. Vis. Sci,* 2012, vol. 53, 2804-2811
- **RUZSICS Z ; WAGNER M ; OSTERLEHNER A ; COOK J ; KOSZINOWSKI U ; BURGERT HG.** *J Virol.,* August 2006, vol. 80 (16), 8100-13
- **SHAYAKHMETOV, D. M. ; PAPAYANNOPOULOU, T. ; STAMATOYANNOPOULOS, G. ; LIEBER, A.** Efficient Gene Transfer into Human CD34+ Cells by a Retargeted Adenovirus Vector. *J. Virol.,* 2000
- **FLICKINGER JR JC ; SINGH J ; CARLSON R et al.** Chimeric Ad5.F35 vector evades anti-adenovirus serotype 5 neutralization opposing GUCY2C-targeted antitumor immunity. *Journal for ImmunoTherapy of Cancer.,* 2020
- **RUZSICS Z ; LEMNITZER F ; THIRION C.** *Methods Mol Biol.,* 2014, vol. 1089, 143-58
- **JORDAN I ; VOS A ; BEILFUSS S ; NEUBERT A ; BREUL S ; SANDIG V.** An avian cell line designed for production of highly attenuated viruses. *Vaccine,* 2009, vol. 27, 748-756, https://doi.org/10.1016/j.vaccine.2008.11.066. PMID 19071186
- **JORDAN I ; NORTHOFF S ; THIELE M ; HARTMANN S ; HORN D ; HÖWING K ; BERNHARDT H ; OEHMKE S ; VON HORSTEN H ; REBESKI D.** A chemically defined production process for highly attenuated poxviruses. *Biologicals,* 2011, vol. 39, 50-58, https://doi.org/10.1016/j.biologicals.2010.11.005. PMID 21237672
- **JORDAN I ; HORN D ; JOHN K ; SANDIG V.** A genotype of modified vaccinia Ankara (MVA) that facilitates replication in suspension cultures in chemically defined medium. *Viruses,* 2013, vol. 5, 321-339
- **JORDAN I ; HORN D ; THIELE K ; HAAG L ; FIDDEKE K ; SANDIG V.** A Deleted Deletion Site in a New Vector Strain and Exceptional Genomic Stability of Plaque-Purified Modified Vaccinia Ankara (MVA. *Virol Sin,* 2019, https://doi.org/10.1007/s12250-019-00176-3. PMID 31833037
- **MAENG et al.** *Journal of Clinical Oncology,* 20 May 2019, vol. 37 (15), 2639-2639
- **LIU, Z. et al.** Systematic comparison of 2A peptides for cloning multi-genes in a polycistronic vector. *Sci. Rep.,* 2017, vol. 7
- **RAAB, D. ; GRAF, M. ; NOTKA, F. ; SCHÖDL, T. ; WAGNER, R.** The GeneOptimizer Algorithm: using a sliding window approach to cope with the vast sequence space in multiparameter DNA sequence optimization. *Syst. Synth. Biol.,* 2010, vol. 4, 215-225
- **BOUSSIF, O. et al.** A versatile vector for gene and oligonucleotide transfer into cells in culture and in vivo: polyethylenimine. *Proc. Natl. Acad. Sci.,* 1995, vol. 92, 7297 LP-7301
- **RUZSICS, Z. ; LEMNITZER, F. ; THIRION, C.** Engineering Adenovirus Genome by Bacterial Artificial Chromosome (BAC) Technology BT - Adenovirus: Methods and Protocols. Humana Press, 2014, 143-158

- **KIENER, R. et al.** Vaccine vectors based on Adenovirus 19a/64 exhibit broad cellular tropism and potently restimulate HCMV-specific T cell responses ex vivo. *Sci. Rep.,* 2018, vol. 8, 1474

- **NIELSEN, K. N. ; STEFFENSEN, M. A. ; CHRISTENSEN, J. P. ; THOMSEN, A. R.** Priming of CD8 T cells by adenoviral vectors is critically dependent on B7 and dendritic cells but only partially dependent on CD28 ligation on CD8 T cells. *J. Immunol.,* 2014, vol. 193, 1223-1232

- **CHEN, X. ; ZARO, J. L. ; SHEN, W.-C.** Fusion protein linkers: property, design and functionality. *Adv. Drug Deliv. Rev.,* 2013, vol. 65, 1357-1369

- **HOLST, P. J. et al.** MHC Class II-Associated Invariant Chain Linkage of Antigen Dramatically Improves Cell-Mediated Immunity Induced by Adenovirus Vaccines. *J. Immunol.,* 2008, vol. 180, 3339-3346

- **KIM, J. H. et al.** High cleavage efficiency of a 2A peptide derived from porcine teschovirus-1 in human cell lines, zebrafish and mice. *PLoS One,* 2011, vol. 6

- **LIU, Y. et al.** *Multiple Functions of Human Papillomavirus Type 16 E6 Contribute to the Immortalization of Mammary Epithelial Cells,* 1999, vol. 73, 7297-7307

- **WIEKING, B. G. et al.** A Non-oncogenic HPV 16 E6/E7 Vaccine Enhances Treatment of HPV Expressing Tumors. *Cancer Gene Ther.,* 2013, vol. 19, 667-674

- **EDMONDS, C. ; VOUSDEN, K. H.** *A Point Mutational Analysis of Human Papillomavirus Type 16 E7 Protein,* 1989, vol. 63, 2650-2656

- **ASBACH, B. et al.** Priming with a Potent HIV-1 DNA Vaccine Frames the Quality of Immune Responses prior to a Poxvirus and Protein Boost. *J. Virol.,* 2019, vol. 93

- **SARWAR, U. N. et al.** Safety and immunogenicity of DNA vaccines encoding Ebolavirus and Marburgvirus wild-type glycoproteins in a phase I clinical trial. *J. Infect. Dis.,* 2015, vol. 211, 549-557

- **JOSEPH, S. et al.** A Comparative Phase I Study of Combination, Homologous Subtype-C DNA, MVA, and Env gp140 Protein/Adjuvant HIV Vaccines in Two Immunization Regimes. *Front. Immunol.,* 2017, vol. 8, 149

- **PANTALEO, G. et al.** Safety and immunogenicity of a multivalent HIV vaccine comprising envelope protein with either DNA or NYVAC vectors (HVTN 096): a phase 1b, double-blind, placebo-controlled trial. *Lancet HIV,* 2019, vol. 6, e737-e749

- **BAROUCH, D. H. et al.** A human T-cell leukemia virus type 1 regulatory element enhances the immunogenicity of human immunodeficiency virus type 1 DNA vaccines in mice and nonhuman primates. *J. Virol.,* 2005, vol. 79, 8828-8834

- **FOUGEROUX, C. ; TURNER, L. ; BOJESEN, A. M. ; LAVSTSEN, T. ; HOLST, P. J.** Modified MHC Class II-Associated Invariant Chain Induces Increased Antibody Responses against Plasmodium falciparum Antigens after Adenoviral Vaccination. *J. Immunol.,* 2019, vol. 202, 2320-2331

- **MALCLES, M.-H. ; CUEILLE, N. ; MECHALI, F. ; COUX, O. ; BONNE-ANDREA, C.** Regulation of bovine papillomavirus replicative helicase e1 by the ubiquitin-proteasome pathway. *J. Virol.,* 2002, vol. 76, 11350-11358

- **MECHALI, F. ; HSU, C.-Y. ; CASTRO, A. ; LORCA, T. ; BONNE-ANDREA, C.** Bovine papillomavirus replicative helicase E1 is a target of the ubiquitin ligase APC. *J. Virol.,* 2004, vol. 78, 2615-2619

- **EWAISHA, R. ; PANICKER, G. ; MARANIAN, P. ; UNGER, E. R. ; ANDERSON, K. S.** Serum Immune Profiling for Early Detection of Cervical Disease. *Theranostics,* 2017, vol. 7, 3814-3823

- **DERSH, D. ; YEWDELL, J. W. ; WEI, J.** A SIINFEKL-Based System to Measure MHC Class I Antigen Presentation Efficiency and Kinetics. *Methods Mol. Biol.,* 2019, vol. 1988, 109-122

- **RAGONNAUD, E. et al.** Replication deficient human adenovirus vector serotype 19a/64: Immunogenicity in mice and female cynomolgus macaques. *Vaccine,* 2018, vol. 36, 6212-6222

- **ESPOSITO, I. et al.** MHC class II invariant chain-adjuvanted viral vectored vaccines enhances T cell responses in humans. *Sci. Transl. Med.,* 2020, vol. 12

- **BOUSSIF, O. ; LEZOUALC'H, F. ; ZANTA, M. A. ; MERGNY, M. D. ; SCHERMAN, D. ; DEMENEIX, B. ; BEHR, J. P.** A versatile vector for gene and oligonucleotide transfer into cells in culture and in vivo: polyethylenimine. *Proceedings of the National Academy of Sciences,* 1995, vol. 92 (16), 7297 LP-7301, https://doi.org/10.1073/pnas.92.16.7297

- **KIENER, R. ; FLEISCHMANN, M. ; SCHWEGLER, C. ; RUZSICS, Z. ; THIRION, C. ; SCHRÖDEL, S. ; ASBACH, B. ; WAGNER, R.** Vaccine vectors based on Adenovirus 19a/64 exhibit broad cellular tropism and potently restimulate HCMV-specific T cell responses ex vivo. *Scientific Reports,* 2018, vol. 8 (1), 1474, https://doi.org/10.1038/s41598-018-19874-1

- **BOROWICZ, S. ; VAN SCOYK, M. ; AVASARALA, S. ; KARUPPUSAMY RATHINAM, M. K. ; TAULER, J. ; BIKKAVILLI, R. K. ; WINN, R. A.** The Soft Agar Colony Formation Assay. *JoVE,* 2014, vol. 92, e51998, https://doi.org/doi:10.3791/51998

- **SCHINDELIN, J. ; ARGANDA-CARRERAS, I. ; FRISE, E. ; KAYNIG, V. ; LONGAIR, M. ; PIETZSCH, T. ; PREIBISCH, S. ; RUEDEN, C. ; SAALFELD, S. ; SCHMID, B.** Fiji: an open-source platform for biological-image analysis. *Nature Methods,* 2012, vol. 9 (7), 676-682, https://doi.org/10.1038/nmeth.2019

- **JORDAN, I. ; HORN, D. ; THIELE, K. ; HAAG, L. ; FIDDEKE, K. ; SANDIG, V.** *Virol Sin.,* April 2020, vol. 35 (2), 212-226, 10.1007/s12250-019-00176-3

- **FELTKAMP et al.** *Eur J Immunol,* 1993
- **JANIK et al.** *Cancer Res,* 1975